(19) Europäisches Patentamt / European Patent Office / Office européen des brevets

(11) **EP 3 243 554 B1**

(12) **EUROPEAN PATENT SPECIFICATION**

(45) Date of publication and mention of the grant of the patent:
**20.07.2022 Bulletin 2022/29**

(21) Application number: **15876942.2**

(22) Date of filing: **15.10.2015**

(51) International Patent Classification (IPC):
*A63B 69/00* (2006.01)    *A63B 69/18* (2006.01)
*A63B 71/06* (2006.01)    *G16H 20/30* (2018.01)
*A63B 24/00* (2006.01)    *G01P 3/00* (2006.01)
*G01P 15/00* (2006.01)

(52) Cooperative Patent Classification (CPC):
**G16H 20/30; A63B 69/00; A63B 69/18; A63B 71/06**

(86) International application number:
**PCT/JP2015/079177**

(87) International publication number:
**WO 2016/111069 (14.07.2016 Gazette 2016/28)**

(54) **INFORMATION PROCESSING DEVICE, INFORMATION PROCESSING METHOD, AND PROGRAM**

INFORMATIONSVERARBEITUNGSVORRICHTUNG, INFORMATIONSVERARBEITUNGSVERFAHREN UND PROGRAMM

DISPOSITIF DE TRAITEMENT D'INFORMATIONS, PROCÉDÉ DE TRAITEMENT D'INFORMATIONS, ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2015 JP 2015000414**
         **05.01.2015 JP 2015000412**

(43) Date of publication of application:
**15.11.2017 Bulletin 2017/46**

(73) Proprietor: **Sony Group Corporation**
**Tokyo 108-0075 (JP)**

(72) Inventors:
• **MATSUZAWA, Sota**
**Tokyo 108-0075 (JP)**
• **OGATA, Takashi**
**Tokyo 108-0075 (JP)**
• **OOKUBO, Masashi**
**Tokyo 108-0075 (JP)**

(74) Representative: **MFG Patentanwälte Meyer-Wildhagen Meggle-Freund Gerhard PartG mbB**
**Amalienstraße 62**
**80799 München (DE)**

(56) References cited:
JP-A- H10 340 040     JP-A- 2008 253 470
JP-A- 2009 050 368     JP-A- 2010 273 746
JP-A- 2011 120 644     JP-A- 2012 120 579
US-A1- 2013 044 043     US-A1- 2013 330 054

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present disclosure relates to an information processing device, an information processing method, and a program.

Background Art

A variety of technologies for detecting diverse behaviors of a user on the basis of, for example, sensor data provided by sensors installed on the user have been proposed. For example, Patent Literature 1 discloses an information processing device which has a plurality of behavior determination units that are specialized in specific behaviors among behaviors of a user, which are recognized through processing of threshold values of sensor data, and generates behavior information on the basis of determination results of the respective behavior determination units.

Further, patent application US 2013/0044043 A1 describes a head mounted information system comprising a gyroscope configured to produce angular velocity signals representing a head angular velocity and further detects a jump by the user based on these signals.

Besides, patent application US 2013/330054 A1 discloses a system for automatic digital curation, annotation, and tagging of action videos. The system receives, in a processing system, GPS data and inertial data from a sensor-based device carried by a sportsman during a sporting activity and detects turns performed by the sportsman.

Citation List

Patent Literature

Patent Literature 1: JP 2010-198595A

Disclosure of Invention

Technical Problem

However, since a variety of behaviors (actions) occur in a daily life of a user, the technology disclosed in Patent Literature 1, for example, does not necessarily enable all of the actions of the user to be detected and information regarding the detected actions to be provided.

Therefore, the present disclosure proposes a novel and improved information processing device, information processing method, and program which enable information regarding a wider variety of actions of a user to be provided.

Solution to Problem

According to a first aspect the invention provides an information processing device in accordance with claim 1. According to a second aspect the invention provides an information processing method in accordance with claim 11. According to a third aspect the invention provides a program in accordance with claim 12. Further aspects of the invention are set forth in the dependent claims, the drawings and the following description of embodiments.

Advantageous Effects of Invention

According to the present disclosure described above, information regarding a wider variety of actions of a user can be provided.

Note that the effects described above are not necessarily limitative. With or in the place of the above effects, there may be achieved any one of the effects described in this specification or other effects that may be grasped from this specification.

Brief Description of Drawings

[FIG. 1] FIG. 1 is a block diagram illustrating a schematic functional configuration of an information processing device according to an embodiment of the present disclosure.

[FIG. 2] FIG. 2 is a flowchart showing a first example of a process for detecting a jump included in an action of a user .

[FIG. 3] FIG. 3 is a flowchart showing an example of a high impact detection process shown in FIG. 2.

[FIG. 4] FIG. 4 is a flowchart showing a first example of a free fall detection process shown in FIG. 2.

[FIG. 5] FIG. 5 is a flowchart showing a second example of the free fall detection process shown in FIG. 2.

[FIG. 6] FIG. 6 is a flowchart showing a second example of the process for detecting a jump included in an action of a user.

[FIG. 7] FIG. 7 is a flowchart showing an example of a candidate section detection process shown in FIG. 6.

[FIG. 8] FIG. 8 is a flowchart showing an example of a vertical acceleration calculation process shown in FIG. 7.

[FIG. 9] FIG. 9 is a flowchart showing an example of a horizontal acceleration calculation process shown in FIG. 7.

[FIG. 10] FIG. 10 is a flowchart showing an example of a process for detecting a turn section included in an action of a user .

[FIG. 11] FIG. 11 is a flowchart showing an example of a rotation section detection process shown in FIG. 10.

[FIG. 12] FIG. 12 is a flowchart showing an example of a head shake detection process shown in FIG. 10.

[FIG. 13] FIG. 13 is a chart showing an example of a turn detection process shown in FIG. 10.

[FIG. 14] FIG. 14 is a block diagram illustrating an example of a process for estimating a sensor mounting state .

[FIG. 15] FIG. 15 is a flowchart showing a process of a first example of information generation.

[FIG. 16] FIG. 16 is a diagram showing a screen display in the first example of information generation .

[FIG. 17] FIG. 17 is a flowchart showing a process of a second example of information generation .

[FIG. 18] FIG. 18 is a diagram showing a screen display of the second example of information generation .

[FIG. 19] FIG. 19 is a diagram showing a screen display of the second example of information generation .

[FIG. 20] FIG. 20 is a diagram for describing a concept of a third example of information generation .

[FIG. 21] FIG. 21 is a flowchart showing a process of the third example of information generation .

[FIG. 22] FIG. 22 is a diagram for describing a video processing in the process exemplified in FIG. 21.

[FIG. 23] FIG. 23 is a diagram for describing a user interface for downloading a video in the process shown in FIG. 21.

[FIG. 24] FIG. 24 is a flowchart showing a process of a fourth example of information generation .

[FIG. 25] FIG. 25 is a diagram illustrating a screen display in the fourth example of information generation.

[FIG. 26] FIG. 26 is a block diagram illustrating a hardware configuration example of an information processing device .

[FIG. 27] FIG. 27 is a diagram for describing a calculation of time-series scores for content control .

[FIG. 28] FIG. 28 is a diagram for describing an example of content control using a time-series score.

[FIG. 29] FIG. 29 is a diagram for describing an example of timings defined for an action.

[FIG. 30] FIG. 30 is a diagram illustrating a first example of effects provided at timings defined for actions.

[FIG. 31] FIG. 31 is a diagram illustrating a second example of effects provided at timings defined for actions

[FIG. 32] FIG. 32 is a diagram illustrating a third example of effects provided at timings defined for actions in an embodiment of the present disclosure.

[FIG. 33] FIG. 33 is a diagram illustrating a fourth example of effects provided at timings defined for actions.

[FIG. 34] FIG. 34 is a block diagram illustrating a hardware configuration example of an information processing device.

Mode(s) for Carrying Out the Invention

Hereinafter, examples of the present disclosure will be described in detail with reference to the appended drawings. In this specification and the appended drawings, structural elements that have substantially the same function and structure are denoted with the same reference numerals, and repeated explanation of these structural elements is omitted.

Note that description will be provided in the following order.

1. Functional configuration of information processing device
2. Examples of action detection process
2-1. Detection of jump-1
2-2. Detection of jump-2
2-3. Detection of turn
3. Examples of additional processes
3-1. Calculation of action score
3-2. Clustering process
3-3. Estimation of sensor mounting state
4. Examples of information generation
4-1. First example
4-2. Second example

4-3. Third example

4-4. Fourth example

4-5. Regarding profile of user

5. Hardware configuration

6. Supplement

7. Examples of content control

7-1. Control using time-series score

7-2. Control using timing defined for action

7-3. Regarding profile of user

8. Hardware configuration

9. Supplement

(1. Functional configuration of information processing device)

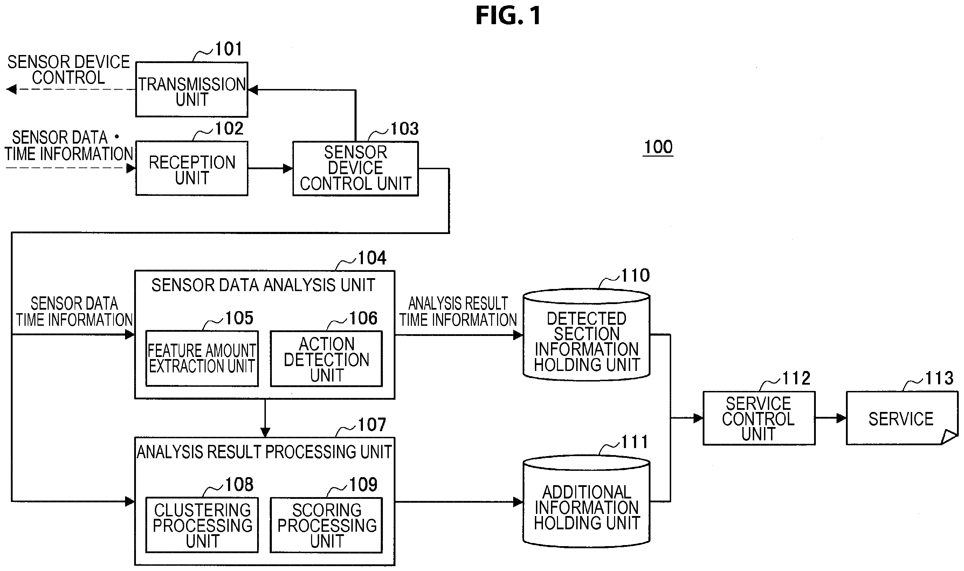

FIG. 1 is a block diagram illustrating a schematic functional configuration of an information processing device according to an embodiment of the present disclosure. Referring to FIG. 1, an information processing device 100 includes a transmission unit 101, a reception unit 102, a sensor device control unit 103, a sensor data analysis unit 104, an analysis result processing unit 107, a detected section information holding unit 110, an additional information holding unit 111, and a service control unit 112.

The information processing device 100 can be, for example, a single device constituting a server on a network or a set of devices as will be introduced in several specific examples to be described below. In addition, the information processing device 100 may be a terminal device that communicates with a server via a network or an independently operating terminal device. Alternatively, functions of the information processing device 100 may be realized by distributing them to a server and a terminal device that communicate with each other on a network. The information processing device 100 or hardware configurations of each of a plurality of devices that realize the information processing device 100 will be described below.

The transmission unit 101 and the reception unit 102 are realized by, for example, communication devices that communicate with a sensor device using various wired or wireless communication schemes. The sensor device includes at least one sensor mounted on a user or a piece of equipment used by the user. The transmission unit 101 transmits control signals output by the sensor device control unit 103 to the sensor device. The reception unit 102 receives sensor data and time information (a timestamp) from the sensor device, and inputs the data into the sensor device control unit 103. In the illustrated example, the reception unit 102 realizes a sensor data reception unit that receives sensor data provided by a sensor mounted on a user or a piece of equipment used by the user. Note that, for example, when the information processing device 100 is a terminal device having at least one sensor, more specifically, a mobile device or a wearable device, the sensor data reception unit may be realized by a processor such as a central processing unit (CPU) that executes a driver program for receiving sensor data from a sensor. In addition, the information processing device according to the present embodiment may have, for example, an acquisition unit that acquires sensor data from an external device having a sensor. Here, the acquisition unit is realized by, for example, a processor such as a CPU that executes "a driver program that receives sensor data from an external device having a sensor via the communication device, which realizes the transmission unit 101 and the reception unit 102, or the like." Note that, when the acquisition unit is provided, the information processing device according to the present embodiment may be configured to include no sensor data reception unit.

The sensor device control unit 103 is realized by, for example, a processor such as a CPU operating in accordance with a program stored in a memory. The sensor device control unit 103 acquires sensor data and time information from the reception unit 102. The sensor device control unit 103 provides the data to the sensor data analysis unit 104 and the analysis result processing unit 107. The sensor device control unit 103 may perform pre-processing on the data when necessary. In addition, the sensor device control unit 103 outputs control signals of the sensor device to the transmission unit 101. In a few embodiments, the sensor device control unit 103 may output the control signals on the basis of feedback on a result of a process of the sensor data analysis unit 104 or the analysis result processing unit 107.

The sensor data analysis unit 104 is realized by, for example, a processor such as a CPU operating in accordance with a program stored in a memory. The sensor data analysis unit 104 executes a variety of analyses using sensor data provided from the sensor device control unit 103. In the illustrated example, the sensor data analysis unit 104 includes a feature amount extraction unit 105 and an action detection unit 106. The feature amount extraction unit 105 extracts various feature amounts from sensor data. The action detection unit 106 detects actions of a user on the basis of the feature amounts extracted from the sensor data by the feature amount extraction unit 105. In the present embodiment, the actions of the user detected by the action detection unit 106 include turns and/or jumps of the user. Furthermore, the action detection unit 106 may detect other actions of the user including walking, running, standing still, moving in a vehicle, and the like. The action of the user can be detected in association with time information (a timestamp) indicating a section in which the action was performed (an action section). The sensor data analysis unit 104 stores analysis results, more specifically, for example, information including action sections of the user detected by the action detection unit 106 in the detected section information holding unit 110. In addition, the sensor data analysis unit 104 provides analysis results to the analysis result processing unit 107.

The analysis result processing unit 107 is realized by, for example, a processor such as a CPU operating in accordance with a program stored in a memory. The analysis result processing unit 107 generates various kinds of additional information to be used by the service control unit 112 in a later stage on the basis of an analysis result of the sensor data analysis unit 104, more specifically, information of the actions of the user detected by the action detection unit 106. In the illustrated example, the analysis result processing unit 107 includes a clustering processing unit 108 and a scoring processing unit 109. For example, when the detected action of the user includes a plurality of actions of the same type, the clustering processing unit 108 may cause the actions to be in clusters on the basis of feature amounts of the actions (which may be feature amounts extracted by the feature amount extraction unit 105 or intermediate feature amounts calculated by the action detection unit 106). In addition, in the same case, the scoring processing unit 109 may calculate scores indicating evaluation of the actions on the basis of the feature amounts. Furthermore, the clustering processing unit 108 and/or the scoring processing unit 109 may calculate new feature amounts on the basis of sensor data provided from the sensor device control unit 103. The analysis result processing unit 107 causes processing results, more specifically, the result of the clustering by the clustering processing unit 108 or information of the scores calculated by the scoring processing unit 109, to be stored in the additional information holding unit 111 together with the time information (the timestamp).

The detected section information holding unit 110 and the additional information holding unit 111 are realized by, for example, various memories or storage devices. The detected section information holding unit 110 and the additional information holding unit 111 temporarily or permanently store information provided from the sensor data analysis unit 104 and the analysis result processing unit 107 as described above. Information stored in the detected section information holding unit 110 and information stored in the additional information holding unit 111 can be associated with each other using, for example, the time information (the timestamp). In addition, the detected section information holding unit 110 and the additional information holding unit 111 may store information regarding each of a plurality of users.

The service control unit 112 is realized by, for example, a processor such as a CPU operating in accordance with a program stored in a memory. The service control unit 112 controls a service 113 using information stored in the detected section information holding unit 110 and/or the additional information holding unit 111. More specifically, the service control unit 112 generates, for example, information to be provided to a user of the service 113 on the basis of information read from the detected section information holding unit 110 and/or the additional information holding unit 111. Here, the information stored in the detected section information holding unit 110 and/or the additional information holding unit 111 includes information regarding to an action of a user detected by the action detection unit 106 included in the sensor data analysis unit 104 as described above. That is, the service control unit 112 realizes an information generation unit that outputs information regarding the action of the user detected by the action detection unit in the illustrated example. Note that, when the information processing device 100 is a server, for example, information output by the service control unit 112 can be transmitted to a terminal device via a communication device. In addition, when the information processing device 100 is a terminal device, for example, the information output by the service control unit 112 can be provided to an output device such as a display, a speaker, or a vibrator included in the terminal device.

(2. Examples of action detection process)

Examples of an action detection process executed in an embodiment of the present disclosure will be described below. In these examples, jumps and turns made when a user snowboards are detected. In a case of snowboarding, for example, a sensor device including an acceleration sensor, an angular velocity sensor, and the like may be mounted directly on a user by being embedded in his or her clothes or incorporated into a wearable terminal device or a mobile terminal device. Alternatively, the sensor device may be mounted in snowboarding goods, for example, a snowboard.

Note that an action detection process executed in the present embodiment is not limited to jumps and turns made while snowboarding, and the action detection process may be executed for, for example, jumps and turns performed in sports other than snowboarding. For example, since jumps and turns are actions that can be commonly performed in a variety of sports, jumps and turns can be detected in a detection process to be described below regardless of the type of sport. In addition, in the action detection process executed in the present embodiment, actions other than jumps and turns may be detected. For example various technologies used in the behavior recognition technology disclosed in JP 2010-198595A or the like can be applied to such an action detection process.

(2-1. Detection of jump-1)

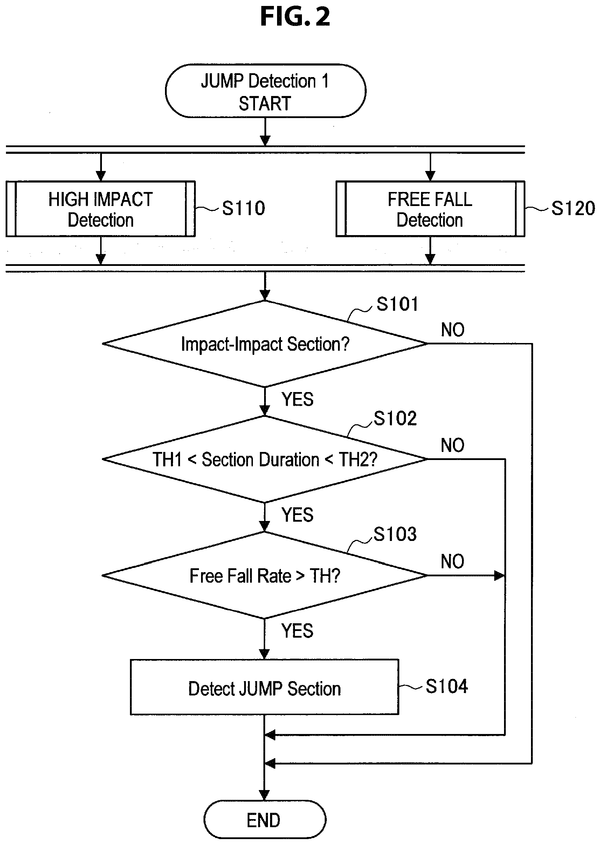

FIG. 2 is a flowchart showing a first example of a process for detecting a jump included in an action of a user in an embodiment of the present disclosure. The illustrated process is executed by, for example, the sensor data analysis unit 104 included in the information processing device 100.

First, the sensor data analysis unit 104 executes a high impact detection process (S110) and a free fall detection process (S120) for each predetermined time frame. Note that these processes will be described in detail below. After receiving results of the processes, the action detection unit 106 included in the sensor data analysis unit 104 determines whether a section sandwiched between two high impact sections has occurred (in which it has been estimated that takeoff and landing are performed) (S101). When such a section has occurred, the action detection unit 106 determines whether duration of the section is between two threshold values (TH1 and TH2) (S102). The threshold values are set, for example, for the purpose of excluding sections that are determined to be too long or too short for a jump.

When the duration is determined to be between the two threshold values in S102, the action detection unit 106 also determines whether a ratio of a free fall section in the aforementioned section exceeds a threshold value (TH) (S103). When the ratio of the free fall section exceeds the threshold value, the section (the section sandwiched between the two high impact sections) is detected to be a jump section (S104).

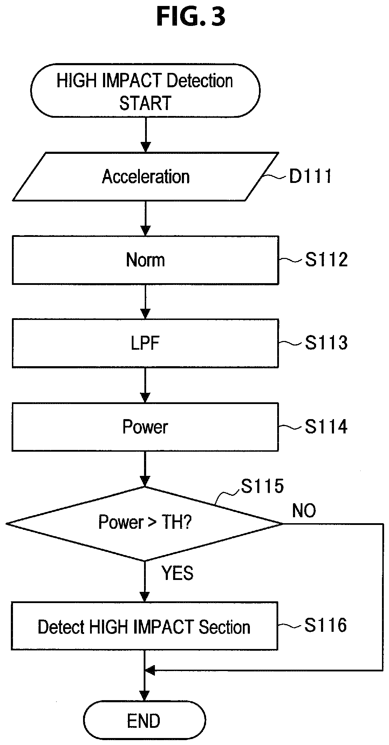

FIG. 3 is a flowchart showing an example of the high impact detection process (S110) shown in FIG. 2. Referring to FIG. 3, acceleration (D111) included in the sensor data is used in the high impact detection process. First, the feature amount extraction unit 105 included in the sensor data analysis unit 104 calculates a norm of acceleration (S112) and then smoothes the norm using a low-pass filter (LPF) (SI13). Next, the feature amount extraction unit 105 calculates a power of amplitude for the norm of acceleration that has undergone smoothing for each predetermined time frame (S114). The action detection unit 106 determines whether the power exceeds a threshold value (TH) (S115), and when the power exceeds the threshold value, the corresponding time frame is detected to be a high impact section (S116).

Note that, in the present specification and drawings, appropriate values are set for threshold values denoted as TH, TH1, TH2, and the like in processes. That is, denoting all threshold values as TH does not mean that all of the threshold values have the same value.

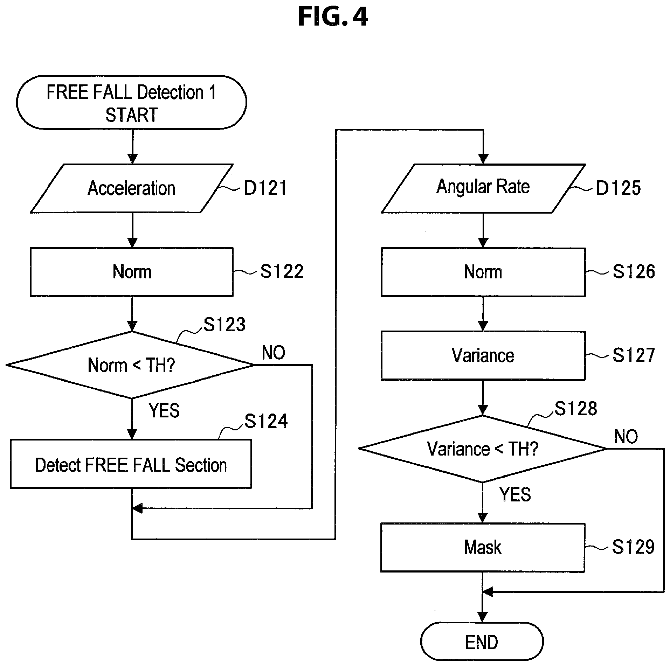

FIG. 4 is a flowchart showing a first example of the free fall detection process (S120) shown in FIG. 2. Referring to FIG. 4, in the free fall detection process as the first example, acceleration (D121) and angular velocity (D125) included in the sensor data are used. First, the feature amount extraction unit 105 calculates a norm of acceleration (S122), and the action detection unit 106 determines whether the norm of each section is lower than a threshold value (TH) (S123). The action detection unit 106 detects a section in which the norm of acceleration is lower than the threshold value to be a free fall section (S124).

Meanwhile, the feature amount extraction unit 105 also calculates a norm of angular velocity (S126), and also calculates a variance of norms in predetermined time frames (S127). The action detection unit 106 determines whether the variance of the norms of angular velocity is lower than a threshold value (TH) (S128), and when the variance is lower than the threshold value, masks the free fall section detected in S124 (i.e., cancels the determination as a free fall section) (S129). The masking process on the basis of the angular velocity is based on the perspective that, since an angular velocity changes when a user makes a jump, a free fall section in which a change (a variance) of an angular velocity is small is caused due to an action other than a jump.

Note that, in the above-described process, the masking process in S126 to S129 may not be necessarily executed after the free fall section determination process in S121 to S124. For example, the action detection unit 106 may first execute the masking process and not execute the free fall section determination process on a section specified as a section to be masked. Alternatively, the masking process may be executed after the jump section detection process (S104) shown in FIG. 2 and a section temporarily detected as a jump section may be masked. Further, the free fall process (S120) shown in FIG. 4 and the like may not be necessarily executed before the section occurrence determination (S101) shown in FIG. 2, and the free fall detection process may be executed on a corresponding section after the section occurrence determination, for example, before a determination of a ratio of a free fall section (S103).

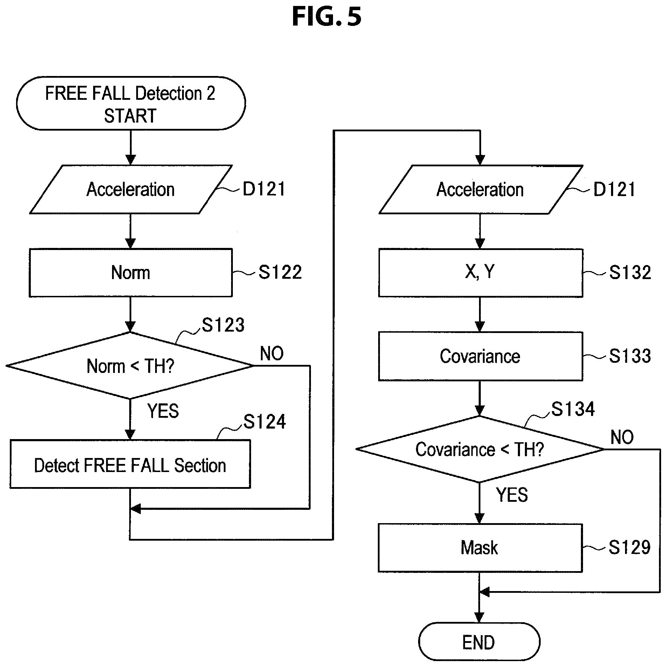

FIG. 5 is a flowchart showing a second example of the free fall detection process (S120) shown in FIG. 2. Referring to FIG. 5, in the free fall detection process as the second example, the acceleration (D121) included in the sensor data provided by the acceleration sensor mounted on a user or a piece of equipment used by the user is used. In S122 to S124, the feature amount extraction unit 105 and the action detection unit 106 execute similar processes to those of the first example and detect a free fall section.

Meanwhile, in the present example, the feature amount extraction unit 105 extracts an X-axis component and a Y-axis component of acceleration (S132), and also calculates covariance of the X-axis component and the Y-axis component of acceleration (S133). More specifically, for example, when a user walks or runs on a reference plane (which is not limited to a horizontal plane and may be a slope), the feature amount extraction unit 105 uses an axis closest to a traveling direction of the user between coordinate axes of an acceleration sensor as the X axis, and an axis closest to a normal direction of the reference plane as the Y axis, and then calculates the covariance of the acceleration components (the X-axis component and the Y-axis component) in axial directions. The action detection unit 106 determines whether the covariance is smaller than a threshold value (TH) (S134) and masks the free fall section detected in S124 when the covariance is smaller than the threshold value (S129). The masking process based on the covariance of the acceleration is effective when a jump desired to be detected is not a so-called vertical jump that only causes displacement in the normal direction of the reference plane, but is a jump that causes displacement in the traveling direction of the user.

(2-2. Detection of jump-2)

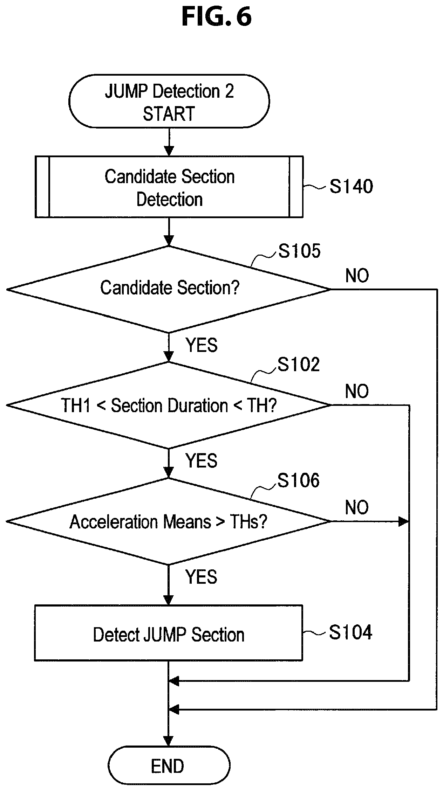

FIG. 6 is a flowchart showing a second example of the process for detecting a jump included in an action of a user in an embodiment of the present disclosure. The illustrated process is executed by, for example, the sensor data analysis unit 104 included in the information processing device 100 as in the first example.

First, the sensor data analysis unit 104 executes a candidate section detection process (S140). Note that details of the process will be described below. After receiving the result of the process, the action detection unit 106 included in the sensor data analysis unit 104 determines whether a candidate section has occurred (S105). When a candidate section occurs, the action detection unit 106 determines whether duration of the section is between two threshold values (TH1 and TH2) (S102) as in the first example. When the duration is between the two threshold values, the action detection unit 106 further determines whether means of acceleration in the vertical direction and the horizontal direction of the section exceed their respective threshold values (THs) (S106). When the means of acceleration exceed their respective threshold value, the candidate section is detected to be a jump section (S104).

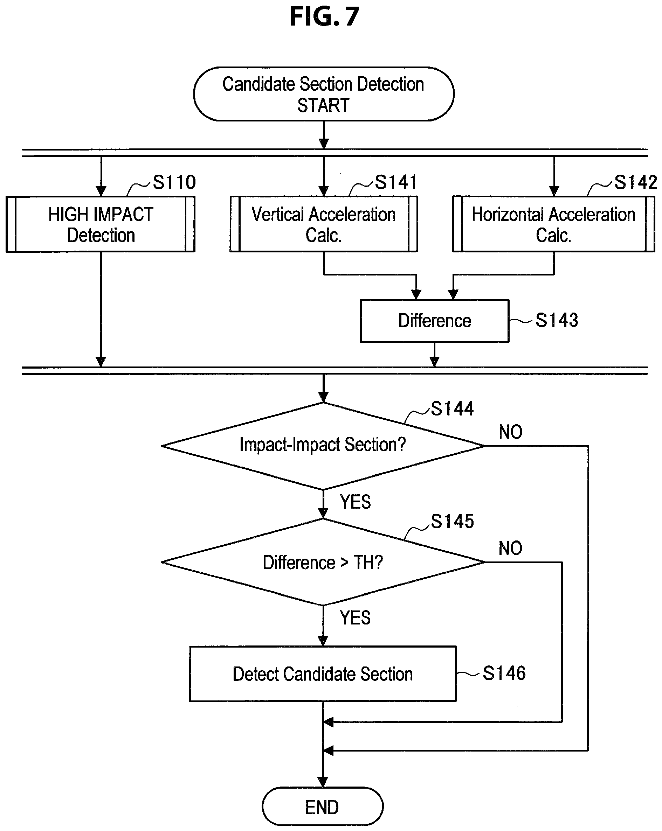

FIG. 7 is a flowchart showing an example of the candidate section detection process (S140) shown in FIG. 6. Referring to FIG. 7, in the candidate section detection process, the high impact detection process (S110) described with reference to FIG. 3 above, a vertical acceleration calculation process (S141), and a horizontal acceleration calculation process (S142) are executed. Furthermore, the feature amount extraction unit 105 included in the sensor data analysis unit 104 calculates a difference between the vertical direction acceleration and the horizontal direction acceleration respectively calculated in S141 and S142 for each section (S143). Then, the action detection unit 106 determines whether a section sandwiched between two high impact sections has occurred (in which takeoff and landing are estimated to be performed) (S144). When such a section has occurred, the action detection unit 106 determines whether a difference between the vertical direction acceleration and the horizontal direction acceleration calculated in S143 exceeds a threshold value (TH) in the section (S145). When the difference exceeds the threshold value, the section (the section sandwiched between two high impact sections) is determined to be a candidate jump section (S146).

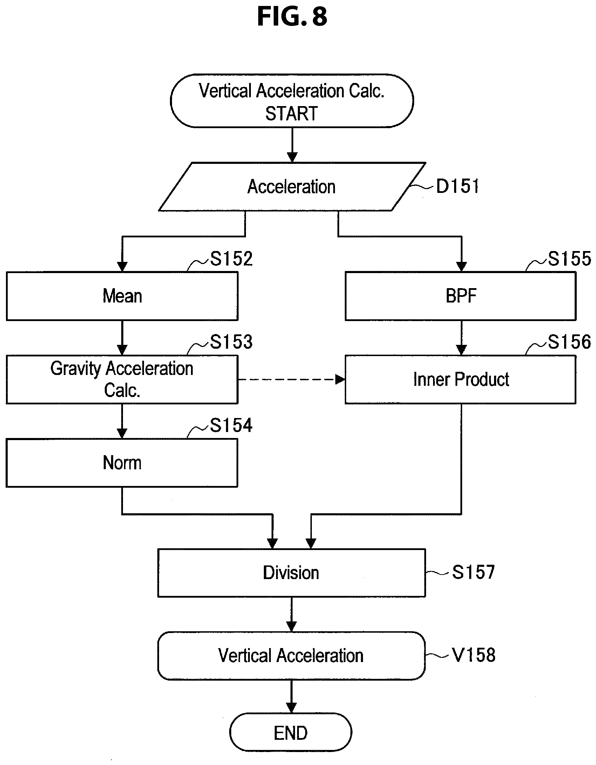

FIG. 8 is a flowchart showing an example of the vertical acceleration calculation process (S141) shown in FIG. 7. Referring to FIG. 8, in the vertical acceleration calculation process, acceleration (D151) included in the sensor data is used. First, the feature amount extraction unit 105 included in the sensor data analysis unit 104 calculates the mean of acceleration (a mean) (S152). The mean calculated here can be, for example, a moving average. On the basis of the mean of the acceleration calculated in S152, the feature amount extraction unit 105 executes a gravitational acceleration calculation process (S153). Further, the feature amount extraction unit 105 calculates a norm of the calculated gravitational acceleration (S154). Note that the gravitational acceleration may be calculated on the basis of the mean such as the moving average or by using a filter such as an LPF.

Meanwhile, the feature amount extraction unit 105 processes the acceleration (D151) with a band-pass filter (BPF) separately from the processes of S152 to S154 (S155). In the illustrated example, the BPF is used for the purpose of removing DC components (i.e., gravity components) included in the acceleration with a low frequency band filter and also performing smoothing on the acceleration with a high frequency band filter. Note that the BPF of S155 may be replaced with a combination of other types of filters, for example, an LPF, a high-pass filter (HPF), and the like. The feature amount extraction unit 105 calculates an inner product of the acceleration processed by the BPF and the gravitational acceleration calculated in S153 (S156).

Further, the feature amount extraction unit 105 divides the inner product calculated in S156 by the norm of the gravitational acceleration calculated in S154 (S157). Accordingly, a vertical acceleration (V158) is obtained. In the illustrated example, the vertical acceleration is calculated by projecting an acceleration obtained by removing a gravitation component with the BPF (S155) in a direction of the gravitational acceleration.

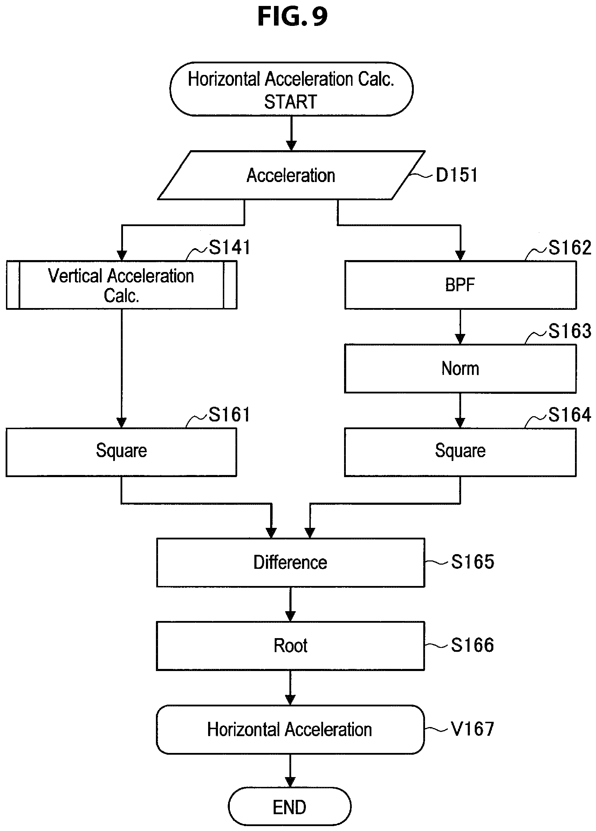

FIG. 9 is a flowchart showing an example of the horizontal acceleration calculation process (S142) shown in FIG. 7. Referring to FIG. 9, the acceleration (D151) included in the sensor data is also used in the horizontal acceleration calculation process. In addition, in the horizontal acceleration calculation process, the vertical acceleration calculated in the vertical acceleration calculation process (S141) described with reference to FIG. 8 is used. More specifically, the feature amount extraction unit 105 included in the sensor data analysis unit 104 squares the vertical acceleration for use (S161).

On the other hand, the feature amount extraction unit acceleration (D151) is processed with the BPF (S162) to remove DC components included in the acceleration and smooth the acceleration. Note that the BPF of S162 may also be replaced with a combination of other types of filters, for example, an LPF, an HPF, and the like. The feature amount extraction unit 105 calculates a norm of the acceleration processed with the BPF (S163) and squares the norm (S164). Further, the feature amount extraction unit 105 calculates a difference between the square of the vertical accel-

7

eration calculated in S161 and the square of the horizontal acceleration calculated in S164 (S165), and obtains the horizontal acceleration (V167) with the difference of the square root (S166).

For the jump detection according to an embodiment of the present disclosure as described above, a total of 3 types of jump detection processes are possible: employing the first example (FIG. 4) of the free fall detection process in the first example (FIG. 2) of jump detection, employing the second example (FIG. 5) of the free fall detection process in the same first example (FIG. 2) of jump detection, and employing the second example (FIG. 6) of jump detection. The sensor data analysis unit 104 that includes the action detection unit 106 may execute each of the three types of jump detection processes and finally detect a jump section on the basis of results of the processes. More specifically, for example, when at least one jump section is detected in the three types of jump detection process, the action detection unit 106 may detect the section as a final jump section. Alternatively, when two or more jump sections are detected, or when all three types of jump section are detected, the action detection unit 106 may detect the sections as final jump sections.

(2-3. Detection of turn)

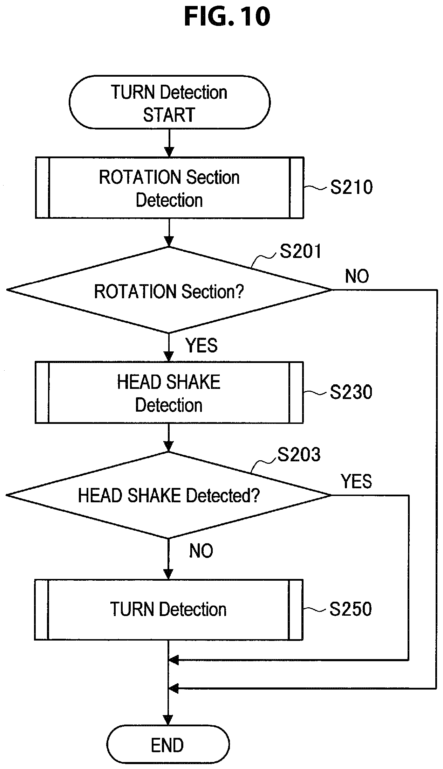

FIG. 10 is a flowchart showing an example of a process for detecting a turn section included in an action of a user in an embodiment of the present disclosure. The illustrated process is executed by, for example, the sensor data analysis unit 104 included in the information processing device 100. In the following processes, the sensor data analysis unit 104 detects rotation included in an action of a user (S210), also detects non-turning rotation included in the rotation (S230), and detects a turn from the rotation from which the non-turning rotation has been removed (S250).

Here, the non-turning rotation includes a rotation occurring through a head shake of the user when, for example, a sensor includes a sensor mounted on the head of the user or equipment mounted on the head of the user. The non-turning rotation can also include a rotation occurring through a body motion, more specifically, a rotation occurring through arm-shaking or arm-circling of the user when a sensor includes a sensor mounted on an arm of the user or a piece of equipment mounted on the arm of the user.

In the present embodiment, a turn section can be detected with higher accuracy by the sensor data analysis unit 104 excluding such a non-turning rotation and then detecting the turn section. From that perspective, the non-turning rotation can be said as noise with respect to a turn to be detected, and in the present embodiment, the sensor data analysis unit 104 can also be said to detect a rotation included in an action of the user, detect noise included in the rotation, and detect a turn from the rotation from which noise has been removed.

First, the sensor data analysis unit 104 executes a rotation section detection process (S210). In the present embodiment, a rotation section is defined to be a section in which an angular velocity in a horizontal plane direction exceeds a threshold value. The sensor data analysis unit 104 determines whether a rotation section has occurred (S201). When a rotation section has occurred, the sensor data analysis unit 104 first executes a head shake detection process (S230). Further the sensor data analysis unit 104 determines whether a head shake has been detected (S203), and when no head shake has been detected, further executes a turn detection process (S250). Through these processes, a section in which a head shake occurs (e.g., a section occurring when a sensor is mounted on a head-mounted wearable terminal device or the like) of the user can be removed from the rotation section, and thus a turn section whose rotation radius, angular velocity, duration, and the like satisfy predetermined conditions can be extracted.

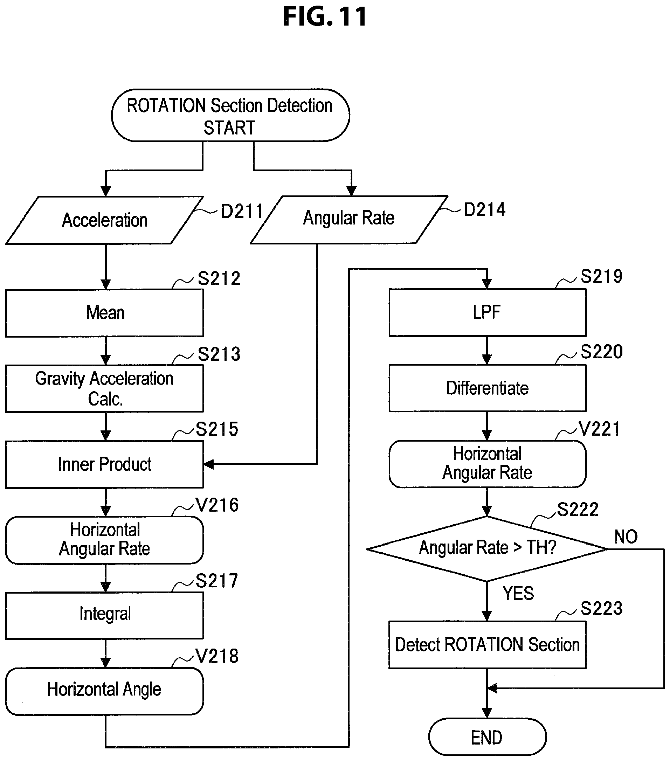

FIG. 11 is a flowchart showing an example of the rotation section detection process (S210) shown in FIG. 10. Referring to FIG. 11, in the rotation section detection process, acceleration (D211) and an angular velocity (D214) included in the sensor data are used. First, the feature amount extraction unit 105 included in the sensor data analysis unit 104 calculates the mean of the acceleration (S212). Here, the calculated mean can be, for example, a moving average. The feature amount extraction unit 105 executes a gravitational acceleration calculation process on the basis of the mean of the acceleration calculated in S212 (S213). Further, the feature amount extraction unit 105 calculates an inner product of a gravitational acceleration calculated in S213 and the angular velocity (D214) (S215). Accordingly, projection of an angular velocity in a direction of the gravitational acceleration, i.e., an angular velocity (V216) in the horizontal plane direction (around a vertical axis) is obtained.

Here, the feature amount extraction unit 105 first integrates the calculated angular velocity (S217), and calculates an angular displacement (V218) in the horizontal plane direction. The feature amount extraction unit 105 processes the angular displacement with a LPF (S219). Further, the feature amount extraction unit 105 differentiates the angular displacement (S220), thereby obtaining an angular velocity in a horizontal plane direction (V221). As the angular velocity of V221 is first integrated in S217 and the angular displacement after the integration is processed with the LPF in S219, the angular velocity of V221 is smoothed in comparison to an angular velocity of V218, and thus noise is removed from waveforms thereof. The action detection unit 106 included in the sensor data analysis unit 104 determines whether the angular velocity (V221) in the horizontal plane direction exceeds a threshold value (S222), and a section in which the angular velocity direction exceeds the threshold value is detected as a rotation section (S223).

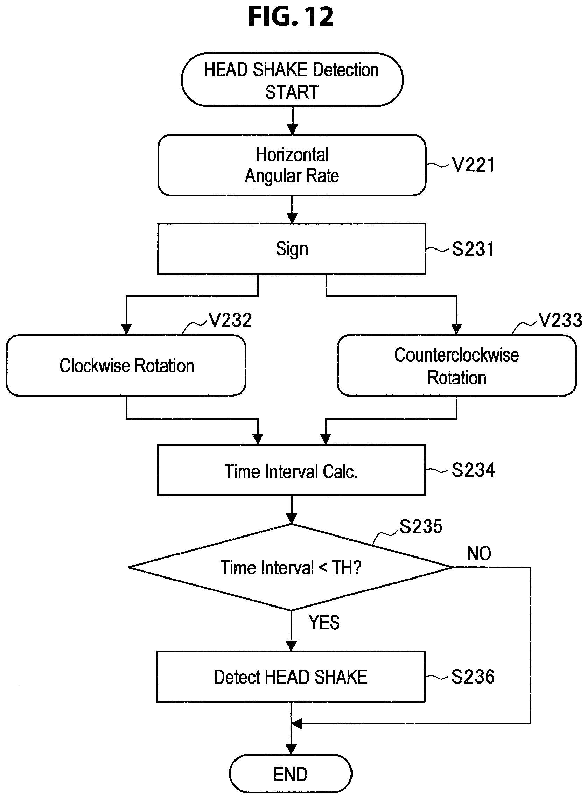

FIG. 12 is a flowchart showing an example of the head shake detection process (S230) shown in FIG. 10.

Referring to FIG. 12, in the head shake detection process, the smoothed angular velocity (V221) in the horizontal plane direction calculated in the rotation section detection process shown in FIG. 11 is used. The feature amount extraction unit 105 acquires a sign of the angular velocity (S231). Although any definition of a sign with respect to a direction of a rotation may be possible, a clockwise rotation (V232) and a counterclockwise rotation (V233) are defined as the sign of the angular velocity (V221) in the illustrated example. Further, the feature amount extraction unit 105 calculates a time interval at which a rotation in a reverse direction has occurred (S234). That is, in the illustrated example, the feature amount extraction unit 105 calculates a time interval from the occurrence of the clockwise rotation (V232) to the occurrence of the counterclockwise rotation (V233) and a time interval between the occurrence of the counterclockwise rotation (V233) and the occurrence of the clockwise rotation (V232). The action detection unit 106 determines whether each time interval calculated in S234 is shorter than a threshold value (TH) (S235), and when each time interval is shorter than the threshold value, the occurrence of a head shake is detected (S236).

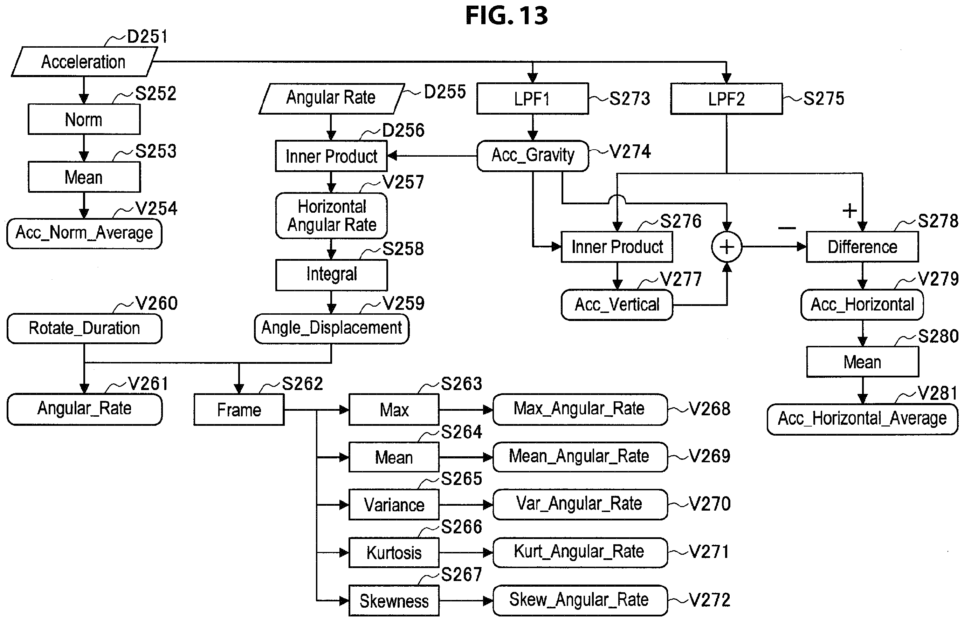

FIG. 13 is a chart showing an example of the turn detection process (S250) shown in FIG. 10. In the turn detection process, the feature amount extraction unit 105 calculates a plurality of feature amounts, and then the action detection unit 106 executes a determination using a threshold value on the basis of the plurality of feature amounts. In FIG. 13, processes of the feature amount extraction unit 105 for calculating each of the feature amounts are shown. Note that, although the processes for calculating each of the feature amounts are described in order below, the processes performed by the feature amount extraction unit 105 may not necessarily be executed in the described order, and can be executed in an arbitrary order as long as prerequisite amounts are acquired or calculated.

First, the feature amount extraction unit 105 calculates a norm of acceleration (D251) included in the sensor data (S252), and calculates the mean of the norm in a predetermined time frame (S253). The average of the norm of acceleration (V254) calculated as described above is used as one of feature amounts for detecting a turn.

Meanwhile, the feature amount extraction unit 105 processes the acceleration (D251) with a first LPF (S273), and calculates gravitational acceleration (V274). Further, the feature amount extraction unit 105 calculates an inner product of an angular velocity (D255) included in the sensor data and the gravitational acceleration (S256). Accordingly, projection of the angular velocity in the direction of the gravitational acceleration, i.e., an angular velocity (V257) in the horizontal plane direction (around the vertical axis), is obtained. The feature amount extraction unit 105 integrates the calculated angular velocity (S258), and calculates angular displacement in a horizontal plane direction (V259). The angular displacement (V259) is also used as one of feature amounts for detecting a turn.

Further, the feature amount extraction unit 105 calculates an angular velocity (V261) on the basis of the angular displacement (V259) and a duration (V260) of a rotation section to be processed. The angular velocity V261 can be smoothed in a longer time frame than the angular velocity D255, (for example, in the entire rotation section. The duration (V260) of the rotation section and an angular change rate (V261) are also used as one type of feature amount for detecting a turn.

In addition, the feature amount extraction unit 105 calculates several feature amounts by analyzing the angular displacement (V259) for a predetermined time frame (S262). More specifically, the feature amount extraction unit 105 calculates a maximum value (S263 and V268), a mean (S264 and V269), a variance (S265 and V270), a kurtosis (S266 and V271), and skewness (S267 and V272) of the angular velocity within the time frame. These feature amounts are also used as feature amounts for detecting a turn.

Meanwhile, the feature amount extraction unit 105 processes the acceleration (D251) with a second LPF (S275). In the illustrated example, while the first LPF (S273) is used to extract the gravitational acceleration (V274) that is a DC component included in the acceleration, the second LPF (S275) is used to smooth the acceleration by filtering out its high frequency area. Thus, pass bands of the LPFs can be set to be different.

The feature amount extraction unit 105 calculates an inner product of the acceleration smoothed by the second LPF (S275) and the gravitational acceleration (V274) extracted by the first LPF (S273) (S276). Accordingly, vertical acceleration (V277) is obtained. Further, the feature amount extraction unit 105 calculates a difference between an acceleration vector composed of the gravitational acceleration (V274) and the vertical acceleration (V277) and the acceleration smoothed by the second LPF (S275) (S278). Accordingly, horizontal acceleration (V279) is obtained. The feature amount extraction unit 105 calculates a mean of horizontal acceleration (S280). The mean of horizontal acceleration (V281) calculated as described above is also used as a feature amount for detecting a turn.

The action detection unit 106 determines whether a turn has occurred on the basis of, for example, the feature amounts extracted from the sensor data as described above. In the illustrated example, the action detection unit 106 executes the determination on the basis of the duration (V260) of the rotation section, the angular displacement (V259) in the horizontal plane direction, the smoothed angular velocity (V261), the mean of the norm of acceleration (V254), the average of the horizontal acceleration (V281), and the maximum value, the mean (V269), the variance (V270), the kurtosis (V271), and the skewness (V272) of the angular velocity within the time frame (V268).

Note that feature amounts to be used in the determination are not limited to the above examples, and, for example, feature amounts other than the above examples may be used or some of the feature amounts of the above example may not be used. For example, types of feature amounts to be used in detection of a turn may be decided from various types of feature amounts that can be extracted from sensor data using main component analysis based on the sensor data obtained when the turn has actually occurred. Alternatively, feature amounts to be used in the determination may be decided on the basis of a propensity of sensor data appearing when a turn has actually occurred. Among the above-described examples, the average of the norm of acceleration (V254) and the average of the horizontal acceleration (V281) are, for example, feature amounts relating to a rotation radius of a turn.

In addition, a threshold value of each feature amount applied to determination by the action detection unit 106 is decided in accordance with, for example, a result of machine learning based on the sensor data obtained when a turn has actually occurred. At this time, whether a turn has actually occurred may be manually decided with reference to, for example, a video of an action simultaneously acquired with the sensor data. Furthermore, a label indicating a type of turn as well as whether a turn has occurred may be given. More specifically, for example, a service provider may give labels that each indicate attributes to an action that is desired to be detected as a turn, desired not to be detected as a turn, or determined to be either or both as a result of referring to a video.

Several examples of the action detection process executed in an embodiment of the present disclosure have been described above. As has already been described, execution of the action detection process in the present embodiment is not limited to jumps and turns occurring during snowboarding, and the action detection process may be executed for jumps and turns occurring in, for example, sports other than snowboarding or scenes other than sports. In addition, an action other than a jump or a turn may be detected in the action detection process executed in the present embodiment. As an example, the action detection unit 106 may detect toppling that occurs in snowboarding or the like. In this case, the feature amount extraction unit 105 may calculate a norm of acceleration similarly to the above-described detection of a jump or a turn, and when the norm of acceleration exceeds a threshold value (e.g., which may be high enough not to appear in normal sliding), the action detection unit 106 may detect the occurrence of toppling.

(3. Examples of additional processes)

(3-1. Calculation of action score)

The scoring processing unit 109 included in the analysis result processing unit 107 calculates, for example, a score for evaluating an action that has occurred (an action score) for an action section including a jump section and/or a turn section detected through the processes described above with reference to FIGS. 2 to 13. The action score can be calculated by, for example, extracting physical amounts (feature amounts) indicating a quality or characteristics of an action from sensor data for an action section and weighting and adding them. The service control unit 112 generates information regarding the action (e.g., a jump or a turn) on the basis of the score calculated as described above.

With respect to a jump section, for example, duration of the section, angular displacement around the X axis/Y axis/Z axis for the section), a ratio of a free fall section, a magnitude of an impact at the time of takeoff/landing, and the like can be extracted as feature amounts for calculating a score. In addition, with respect to a turn section, for example, duration of the section, a displacement angle, a mean, a maximum value, and a standard deviation of a speed, a maximum value and a standard deviation of an angular velocity, and the like can be extracted as feature amounts for calculating a score.

Note that a coefficient of the weighting and addition can be set, for example, in accordance with a property of an action emphasized in the service 113 provided by the information processing device 100. In addition, a method for calculating an action score using feature amounts is not limited to the weighting and addition, and other computation methods may be used. For example, an action score may be calculated by applying a machine learning algorithm such as a linear regression model.

(3-2. Clustering process)

Further, the clustering processing unit 108 included in the analysis result processing unit 107 applies a clustering algorithm, such as a k-means method using feature amounts and the like that are extracted for scoring, to action sections including jump sections and/or turn sections, which are detected through the processes described above with reference to FIGS. 2 to 13, and classifies the detected actions into clusters. In a case of jump sections or turn sections, for example, the actions may be classified into clusters on the basis of a length of duration of sections or a magnitude of rotation. The result of clustering is used to extract action sections so that actions, such as various types of jumps or turns, are included in a dynamic image when, for example, a digest dynamic image is provided as a service. In addition, classifying satisfactory and unsatisfactory actions into different clusters will help a user review his or her actions or can be used in coaching for improving actions.

Note that the analysis result processing unit 107 may compute a degree of similarity of action sections on the basis of a correlation coefficient of feature amounts as a similar process to clustering (action sections having a high degree of similarity can be treated in a similar manner to action sections classified into the same cluster). In addition, for example, the analysis result processing unit 107 may prepare feature amount patterns of actions of typical types in advance and determine to what type of action a newly generated action corresponds.

(3-3. Estimation of sensor mounting state)

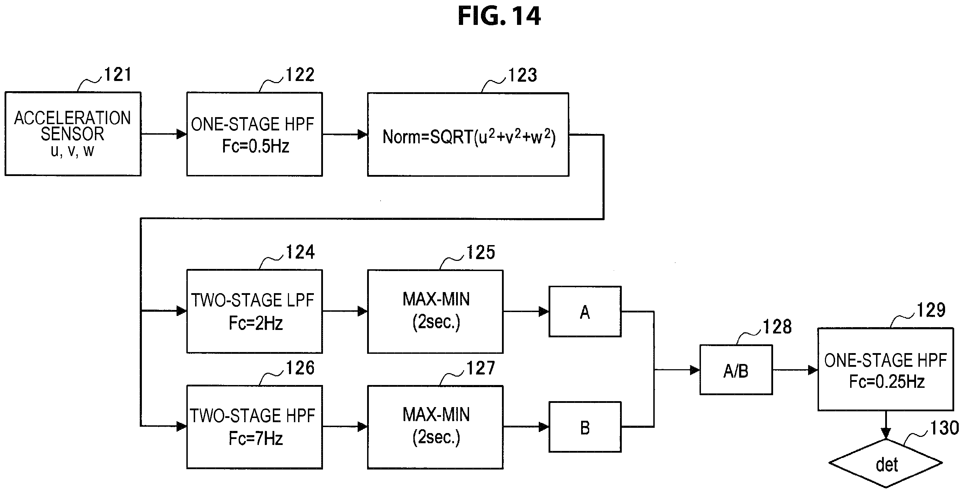

FIG. 14 is a block diagram illustrating an example of a process for estimating a sensor mounting state in an embodiment of the present disclosure. More specifically, the illustrated configuration determines whether a sensor that provides sensor data is mounted directly on the body of a user or mounted on a piece of equipment used by the user. The process shown is executed by, for example, the sensor data analysis unit 104 included in the information processing device 100. Note that, although specific cutoff frequencies (Fc) of filters and lengths of time frames are described in the illustrated example, these numerical values are merely examples and can be appropriately changed in accordance with actual characteristics of a sensor.

In the illustrated example, the reception unit 102 of the information processing device 100 receives sensor data provided by an acceleration sensor 121 with three axes (u, v, and w). The sensor data analysis unit 104 acquires the sensor data via the sensor device control unit 103. The sensor data analysis unit 104 first processes acceleration included in the sensor data with a one-stage HPF 122 (Fc = 0.5 Hz) and then executes a norm calculation 123. Further, using results obtained by processing the norm with a two-stage LPF 124 (Fc = 2 Hz) and a two-stage HPF (Fc = 7 Hz), the sensor data analysis unit 104 calculates amplitudes (differences between maximum values and minimum values) in a 2-second time frame (125 and 127). Using results (A and B) thereof, A/B is computed (128). The result of the computation is processed with a one-stage HPF 129 (Fc = 0.25 Hz), and then threshold determination 130 is executed.

The above-described determination process is based on attenuation of high frequency components of acceleration as the body of a user functions as a LPF when the sensor is mounted directly on the body of the user. A (the amplitude of a low frequency component that has passed the LPF 124)/ B (the amplitude of a high frequency component that has passed the HPF) of the above example has a greater value as a high frequency component of original acceleration attenuates more. Thus, in the threshold determination 130, when a value obtained by processing A/B with the HPF 129 is greater than a threshold value, the sensor can be determined to be mounted directly on the body of the user, and when it is not, the sensor can be determined to be mounted on a piece of equipment.

The result of the above-described estimation may be used in, for example, the sensor data analysis unit 104. In this case, the sensor data analysis unit 104 may change the threshold value, values set for the filters, and the like on the basis of whether the sensor is mounted on a body or a piece of equipment in the user action detection process described above. Alternatively, the result of the above-described estimation may be fed back to the sensor device control unit 103 to be used for setting parameters and the like with respect to a measurement of the sensor device or deciding a pre-processing method for sensor data by the sensor device control unit 103 or the like.

In the present example, processes of sensor data may be adaptively controlled on the basis of an estimation of a state of a sensor data provision side, like, for example, the estimation of a sensor mounting state described above. As another example, the sensor data analysis unit 104 may estimate the type of sport in which an action has occurred using an algorithm such as machine learning from intensity of an impact, a pattern of motion, or the like detected by the acceleration sensor or the like. A sport may be estimated for each event that is generally recognized, or for each category such as board sports, water sports, cycling, motor sports, or the like. In addition, for example, when a sensor is mounted on a piece of equipment, the sensor data analysis unit 104 may estimate the type of equipment (e.g., in a case of skiing, whether the sensor is mounted on a ski or a ski pole). A result of the estimation may be used in, for example, control of a threshold, or values set for the filters for detecting an action, like the result of the estimation of a sensor mounting state, may be fed back to the sensor device control unit 103 to be used for controlling the sensor device or deciding a pre-processing method of sensor data.

(4. Examples of information generation)

Several examples of information generation included in an embodiment of the present disclosure will be described below.

(4-1. First example)

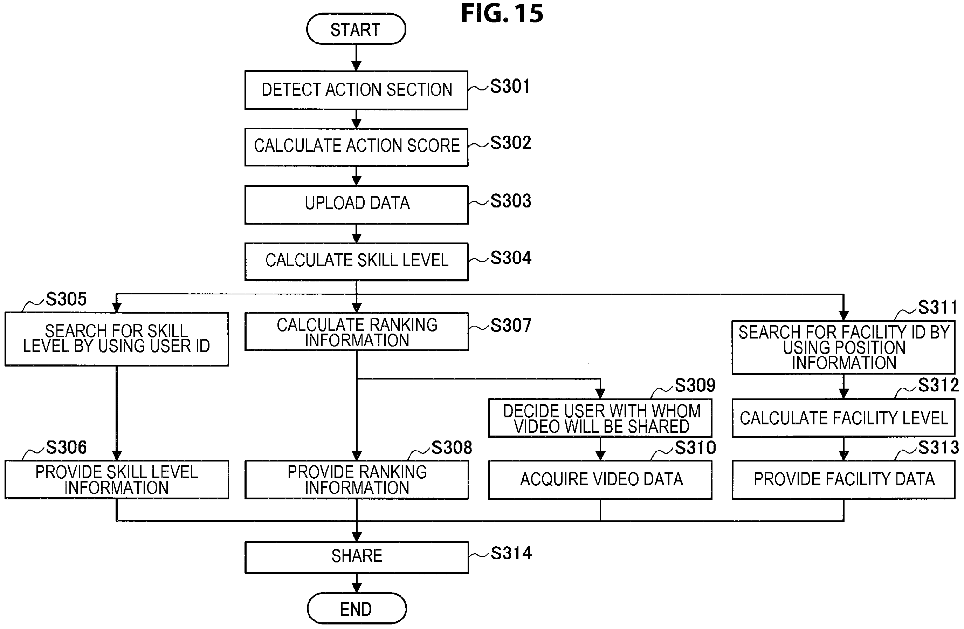

FIG. 15 is a flowchart showing a process of a first example of information generation included in an embodiment of the present disclosure

In the illustrated example, first, the action detection unit 106 included in the sensor data analysis unit 104 detects an action section (S301). The action section may include, for example, a jump section and/or a turn section detected through the processes described above with reference to FIGS. 2 to 13. In addition, the action section may include a section in which another action of a user to be detected on the basis of sensor data, such as walking, running, standing still, moving in a vehicle, or the like, has occurred.

Next, the scoring processing unit 109 included in the analysis result processing unit 107 calculates an action score for the action section detected in S301 (S302). Further, action information relating to the action section and the action score and data including a user ID, position information, separately acquired video data of the action, and the like are uploaded (S303). The uploading of S303 may be, for example, uploading from a server that realizes the functions of the sensor data analysis unit 104 and the analysis result processing unit 107 to a server that realizes the service control unit 112. Alternatively, the uploading of S303 may be uploading from a terminal device that realizes the functions of the sensor data analysis unit 104 and the analysis result processing unit 107 to the server that realizes the service control unit 112. When such servers or terminal devices are the same, the uploading is differently read as, for example, registration in an internal database.

The service control unit 112 that has received the upload of the action section detected with respect to individual users and the action score in S303 calculates, for example, a skill level of the user (S304). As described later, the skill level is calculated on the basis of, for example, a history of action scores calculated for each user. Thus, in the illustrated example, the server realizing the service control unit 112 can use a database holding histories of action scores of users. In addition, the server may be able to use a database holding skill levels of users, and the service control unit 112 that has calculated the skill level in S304 may update the database for the skill levels.

Using the skill level calculated in S304, the service control unit 112 can execute a few processes. For example, the service control unit 112 may search for a skill level of a user by using an input user ID (S305) and provide information regarding the skill level (S306). In addition, the service control unit 112 may calculate ranking information on the basis of the skill level of a target user (S307) and provide the ranking information (S308). Alternatively, the service control unit 112 may decide a user with whom an action video will be shared on the basis of the ranking information (S309) and acquire video data of the user who is a sharing target (S310). Note that, details of a calculation of ranking information and a process using the calculated ranking information will be described below.

Further, the service control unit 112 searches for an ID of a facility in which an action has been detected, by using position information included in the data uploaded in S303 (S311), calculates a facility level on the basis of distributions of action scores of actions detected in the facility and the skill levels of users who performed the actions (S312), and provides facility data including information regarding the facility level (S313). Note that details of the process of calculating a facility level will be described below. The service control unit 112 may share information which a certain user requested on social media or a website with other users when providing the above-described several types of information (S314).

The information calculated in the example illustrated in FIG. 15 will be described below in more detail. Note that all of the information may not necessarily be calculated, and only a part thereof may be calculated. In addition, other types of information may be calculated in addition to or instead of the information.

(Calculation of skill level)

In the above-described example of FIG. 15, the skill level is calculated on the basis of a history of the action score calculated for each user (S304). As a specific example, a skill level may be decided in accordance with a cumulative value of points calculated using a formula including the following variables.

- Sum of action scores
- Maximum value of the action scores
- Distribution of the action scores
- Variation of types of actions (a result of the above-described clustering can be used)
- Level of a facility in which an action has been detected
- Variation of the facility in which an action has been detected
- Number of detected actions in the latest period
- Total number of detected actions
- Frequency of detected actions (the total number of detected actions/service use periods)
- Level of difficulty of a pattern formed by consecutive actions
- Goal achievement rate

Note that the above-described goal accomplishment rate may be, for example, an achievement rate of a goal set by a user him or herself. Alternatively, a goal based on a current skill level of the user may be automatically set by a service, and the skill level may be raised by accomplishing the set goal. Alternatively, an automatically set goal refers to, for example, a goal that will be achieved by a highly skilled person or a heavy user of a service, and an attempt to achieve the goal itself may be recorded.

(Generation of ranking)

In the above-described example of FIG. 15, a ranking is generated on the basis of skill levels of users (S307). More specifically, the ranking is generated by arranging the skill levels calculated for the users to be in ascending order. A ranking may be generated for, for example, all of the users (an overall ranking), for users included in a range designated by a certain user (a ranking within a friend group), for each attribute of the users such as an area or their age, or for each facility in which an action has been detected. The rankings may be saved in a database of a server and updated in real time when an action is detected.

(Sharing of video)

In the above-described example of FIG. 15, an action video is shared between users selected on the basis of the ranking information (S309 and S310). The action video is generated by, for example, cutting out a portion corresponding to an action section from video data captured by an imaging device such as a digital video camera paired with a sensor device.

Here, the action video to be shared can be obtained by editing action videos generated for a plurality of action sections in units of several seconds to dozens seconds in accordance with a viewing environment. For example, when action videos of a single user are edited, videos having higher action scores or lower action score are extracted, or a video of an action detected in a facility in which users who will view the action video are present may be extracted. In addition, when action videos of a plurality of users are edited, a video of a highly-ranked user may be extracted, a video of a highly-ranked user in a facility in which users who will view the action videos are present may be extracted, a video of a user who belongs to a friend group of the users who will view the action videos may be extracted, a video uploaded in the latest period may be extracted, or a video of another user having a skill level close to that of the users who will view the action videos may be extracted.

In addition, when videos are edited, a single action video may be arranged in a time series manner, a screen may be divided, and a plurality of action videos may be reproduced in parallel thereon or the plurality of videos may be displayed to be transparent and thus superimposed on each other. In addition, a generated action image may not only be shared or disclosed on social media or a website, but may also be displayed on, for example, a public display as an advertisement or may be transmitted to a coach and used to get advice.

(Calculation of facility level)

In the above-described example of FIG. 15, with respect to the facility searched for on the basis of the position information, a facility level is calculated on the basis of distributions of action scores of actions detected in the facility and skill levels of users who have performed actions (S312). When a level of a facility is calculated, for example, a low level (indicating a low level of difficulty) may be given to a facility in which low-scoring actions of many users are detected regardless of their skill levels. On the other hand, a high level (indicating a high level of difficulty) may be given to a facility in which high-scoring actions of users having high skill levels are detected. In addition, detection of an action such as toppling (indicating a failure of an attempted action) may also be reflected on a level of a facility. A level indicating a degree of popularity in accordance with the number of users whose actions are detected can also be calculated without limiting the calculation to a level indicating a level of difficulty as described above.

Note that a facility can have a variety of forms, for example, a course, a court, a field, and the like, depending on a type of sport in which an action occurs. In addition, with respect to an example of skiing, facilities can be defined in various units, like a park including a plurality of courses, a specific jump ramp on a course, and the like. Likewise in other sports, facilities can be defined in various units.

(Example of screen display)

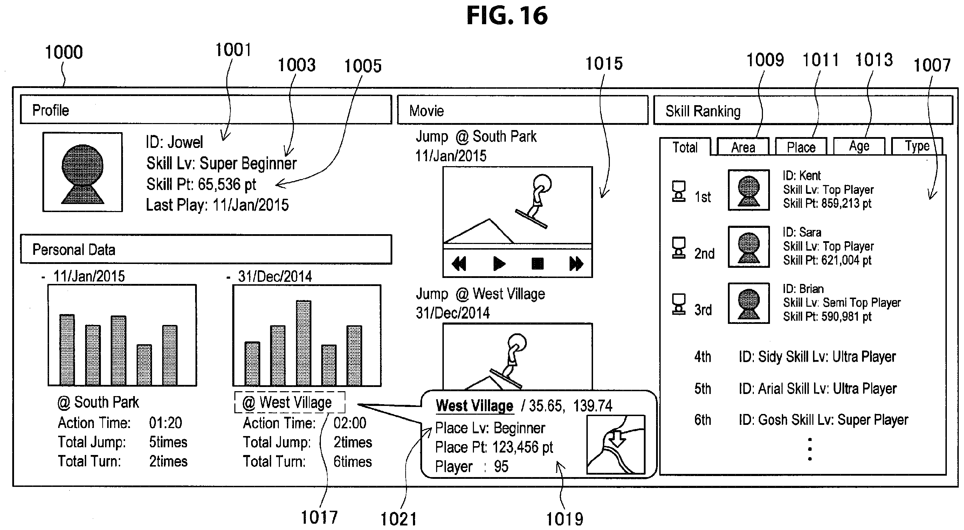

FIG. 16 is a diagram showing a screen display in the first example of information generation included in an embodiment of the present disclosure. Referring to FIG. 16, a screen 1000 displays a user profile 1001. Items of the user profile 1001 include a skill level 1003. The skill level 1003 is, for example, the skill level calculated in the process of S304 shown in FIG. 15. In the illustrated example, the user profile 1001 further includes a skill point 1005. The skill point 1005 may be, for example, a point calculated for deciding a skill level as described above.

In addition, the screen 1000 displays a skill ranking 1007. The skill ranking 1007 is displayed, for example, on the basis of the ranking generated in the process of S307 shown in FIG. 15. In the illustrated example, the skill ranking 1007 can be displayed with regard to a ranking in an area (displayed by selecting a tab 1009), a ranking in a facility (displayed by selecting a tab 1011), a ranking by age (displayed by selecting a tab 1013), and the like in addition to an overall ranking.

Furthermore, the screen 1000 displays a shared action video 1015. The action video 1015 is, for example, a video shared though the processes of S309 and S310 shown in FIG. 15. In addition, the screen 1000 can cause a facility (e.g., a facility name 1017) to be displayed in a pop-up display of facility information 1019. The facility information 1019 includes, for example, a facility level 1021 calculated in the process of S312 shown in FIG. 15.

By disclosing the above-described screen 1000, for example, through social media or web services, mobile devices such as smartphones, applications for wearable devices, and the like, a user can share various kinds of information with other users, check his or her own level, compete with friends, and the like.

(4-2. Second example)

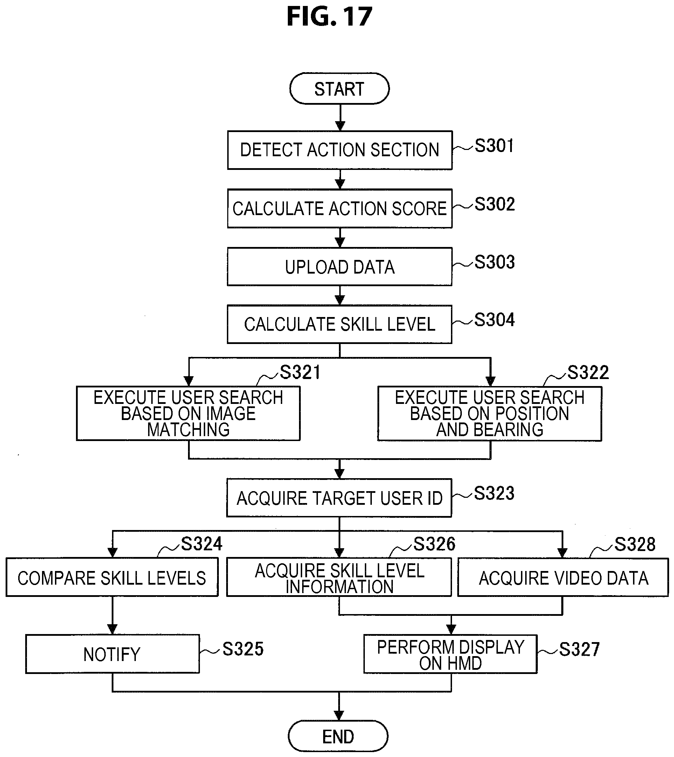

FIG. 17 is a flowchart showing a process of a second example of information generation included in an embodiment of the present disclosure.

In the illustrated example, processes of a detection of an action section (S301), a calculation of an action score (S302), a upload of data (S303), and a calculation of a skill level (S304) are executed as in the first example shown in FIG. 15.

Here, in the present example, the service control unit 112 executes a user search based on image matching (S321), and/or a user search based on a position and a bearing (S322). These processes are processes for searching for another user (a second user) who is in proximity to a user (a first user) who attempts to receive information. In S321, for example, the face of the second user included in a captured image corresponding to a view of the first user (which will also be referred to as a view image hereinbelow) may be detected. Alternatively, in S321, for example, a landmark commonly included in the view image of the first user and a view image of the second user may be detected. In S322, a user is searched for on the basis of position information acquired by a GPS or the like mounted on a terminal device and information of a bearing acquired by a geomagnetic sensor or the like. More specifically, the service control unit 112 may detect the second user as a target user when position information acquired by terminal devices that are respectively carried by the first user and the second user indicates proximity and a bearing in which it is estimated that the first user faces his or her terminal device is close to a bearing of the second user from the first user.

Further, the service control unit 112 acquires a target user ID for the second user searched for in S321 or S322 (S323). The second user, whose target user ID can be acquired, can be, for example, another user who is using the same service as the first user or a service linked to a service being used by the first user. Using the target user ID acquired in S323, the service control unit 112 can execute several processes.

The service control unit 112 compares skill levels, which are calculated in S304, of, for example, the first user who attempts to receive information and the second user that has been detected as the target user (S324). The service control unit 112 transmits a notification to the first user and/or the second user in accordance with a result of the comparison (S325). For example, when an action that occurs in a match-type sport is detected in the present embodiment and the skill level of the first user is close to the skill level of the second user in the comparison of S324, a notification of the presence or proximity of one or both of the users can be transmitted in S325. In this case, a condition that positions of the first user and the second user are in proximity may be set. As will be described below, navigation may be provided such that, in the above case, the users meet each other when the first user and the second user consent thereto.

In addition, for example, the service control unit 112 acquires skill level information of the target second user using the target user ID acquired in S323 (S326). For example, when a terminal device mounted on the first user who attempts to receive information is a glasses-type or a goggle-type head mounted display (HMD), the service control unit 112 causes the acquired skill level information to be displayed on the HMD (S327). In this case, an image displaying the skill level information is displayed, for example, to be superimposed over a real space image on the HMD. Further, for example, the service control unit 112 may acquire data of an action video of the target second user using the target user ID acquired in S323 (S328). The action video of the second user can also be displayed on the HMD mounted on the first user (S327). Note that, when a user is holding a smartphone, a tablet, or the like over his face in a real space, similar information may be displayed using a camera and a display of such a mobile device instead of an HMD.

(Examples of screen display)

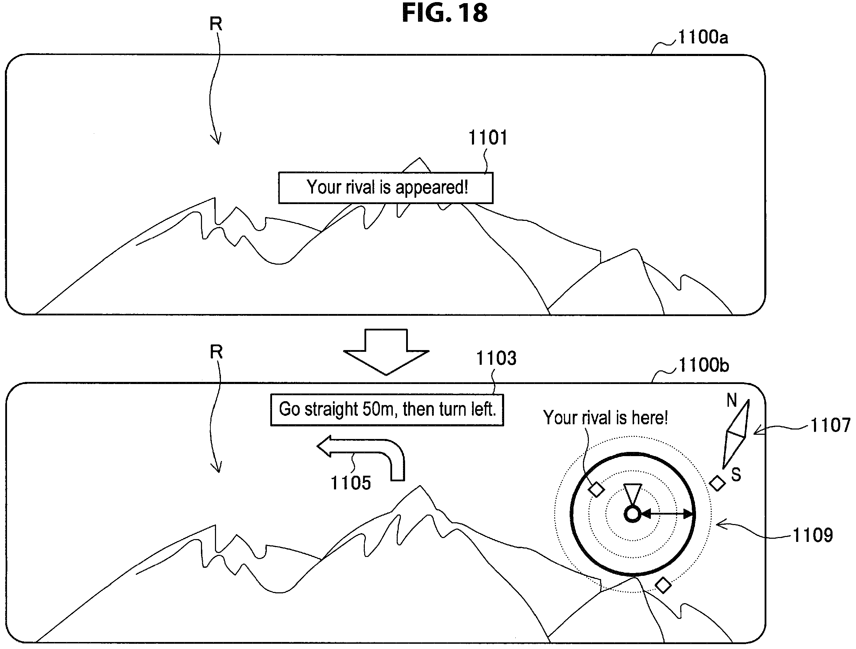

FIG. 18 is a diagram showing a screen display of the second example of information generation included in an embodiment of the present disclosure. Referring to FIG. 18, screens 1100 (screens 1100a and 1100b) are displayed to be superimposed over a real space image R using an HMD. A notification 1101 generated through the above-described process of S325 shown in FIG. 17 is displayed on the screen 1100a. As described above, such a notification 1101 can be displayed when, for example, another user (the second user) having a skill level close to that of a user (the first user) who is viewing the screen 1100 is in proximity with the first user.

In addition, after the notification is displayed on the screen 1100a, for example, consents of the first user and the second user are obtained and navigation is provided on the screen 1100b such that the users can meet each other.

In the illustrated example, the screen 1100b includes instruction text 1103, an arrow 1105, a compass bearing 1107, and a radar map 1109 for navigation.

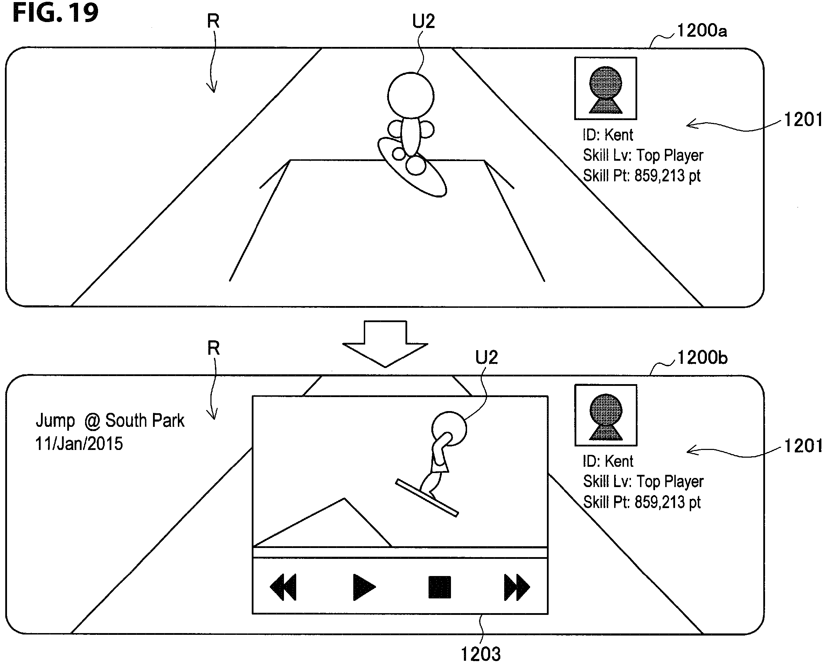

FIG. 19 is also a diagram showing a screen display of the second example of information generation included in an embodiment of the present disclosure. Referring to FIG. 19, screens 1200 (screens 1200a and 1200b) are displayed to be superimposed over the real space image R using an HMD. On the screen 1200a, another user (a second user) U2 is sliding during snowboarding in the real space image R. In the present example, the user U2 is identified on the basis of, for example, an image acquired by a camera mounted on the HMD (S321 shown in FIG. 17). Alternatively, the user U2 may be identified on the basis of a position and a bearing acquired by the HMD and a position (and a bearing) acquired by a terminal device carried by or worn by the user U2 (S322 shown in FIG. 17). In the illustrated example, skill level information 1201 of the identified user U2 is displayed (S326 and S327 shown in FIG. 17). Meanwhile, a past action image 1203 of the user U2 is displayed on the screen 1200b, in addition to the skill level information 1201 of the user U2 (S326, S327, and S328 shown in FIG. 17).

(4-3. Third example)

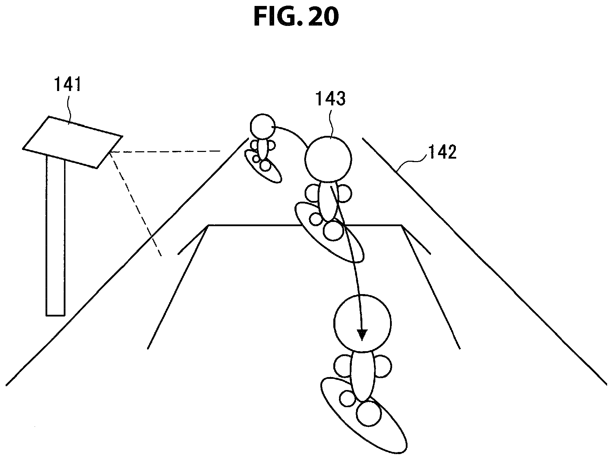

FIG. 20 is a diagram for describing a concept of a third example of information generation included in an embodiment of the present disclosure. Referring to FIG. 20, in a snowboarding course 142 (an example of a facility), a camera 141 installed in the facility is acquiring a video of a user 143 who is sliding during snowboarding. At this time, it is possible for the camera 141 to acquire a video which captures an action of the user 143 that has been performed on the course 142. That is, it is possible for the video acquired by the camera 141 to be useful as a video which captures an action of the user from an objective viewpoint.

However, it is not easy to extract an appropriate portion desired by a user from a video being continuously captured by the camera 141. Thus, in the present example, the service control unit 112 extracts the appropriate portion of the video on the basis of information of an action section or the like. Accordingly, it is possible to provide an appropriate portion of a video desired by a user. Note that, in the present example, the above-described calculation of an action score and a skill level may not necessarily be executed.

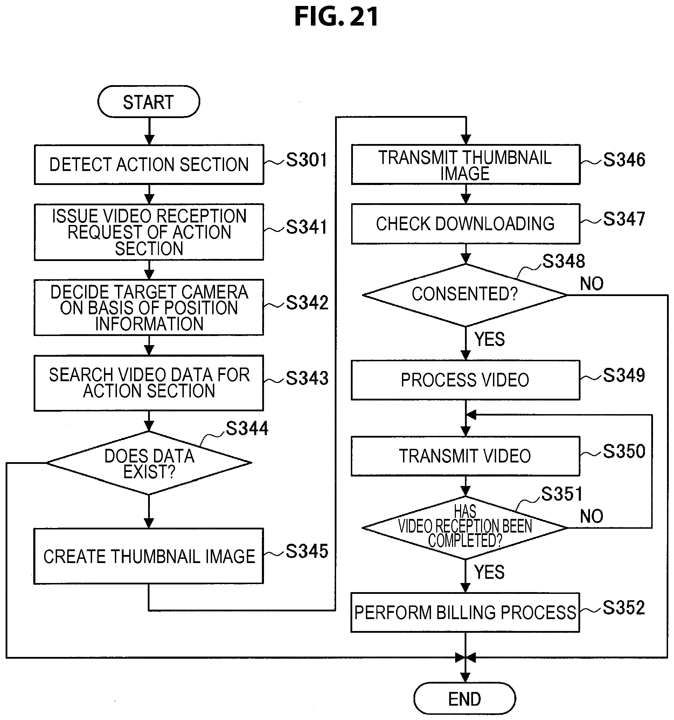

FIG. 21 is a flowchart showing a process of the third example of information generation included in an embodiment of the present disclosure. In the illustrated example, first, the process of a detection of an action section is executed (S301) by the action detection unit 106 included in the sensor data analysis unit 104 similarly to the above-described first and second examples. Next, an action section video reception request is issued (S341). This request may be transmitted, for example, from a server realizing the function of the sensor data analysis unit 104 to a server realizing the service control unit 112. Alternatively, the request of S341 may be transmitted from a terminal device realizing the function of the sensor data analysis unit 104 to a server realizing the service control unit 112. When the servers and terminal devices are the same, issuance of a request can be read as, for example, issuance of an internal command for requesting data. The request can include, for example, information of a target action section, a device ID, and position information indicating a position at which an action occurred.

The service control unit 112 that receives the video reception request in S341 decides a target camera from cameras installed in a facility on the basis of the position information included in the request (S342). The target camera can be, for example, a camera installed at a position closest to a position at which an action indicated by the position information included in the request occurred. Further, the service control unit 112 searches video data acquired by the target camera for an action section included in the request (S343) and determines whether or not data regarding the action section exists (S344). Here, for example, , the service control unit 112 compares a timestamp of the video data and a timestamp of the action section and determines whether or not video data of a time corresponding to the action section exists. Further, the service control unit 112 may execute image analysis on the video data of the action section and determine whether or not a subject making a motion (performing an action) is pictured.

When it is determined that target video data exists in S344, the service control unit 112 further creates a thumbnail image on the basis of the target video data (S345). In the illustrated example, the thumbnail image is created from the target video data by, for example, capturing one or a plurality of still images in the action section. Thus, at this point of time, processing as in S349, which will be described below, may not be applied to the video data. The service control unit 112 transmits the thumbnail image to the terminal device used by the user (S346), and whether or not the user consents to a download of the video is checked in the terminal device (S347). Here, the thumbnail image transmitted to the terminal device is an example of information that notifies the user of the fact that a video obtained by photographing the action section (e.g., a jump section or a turn section) exists. The terminal device determines whether or not the user has consented to the download by an operation being input (S348).

When the user consents to the download of the video in S348, the service control unit 112 processes the video (S349). More specifically, the service control unit 112 cuts a section corresponding to the action section out of the target video data. Further, the service control unit 112 may execute down-converting or the like on the video data in accordance with performance of the terminal device to which the video will be provided (resolution of a display, processing performance of a processor, a communication speed, etc.). When the processing is finished, the service control unit 112 transmits the processed video to the terminal device used by the user (S350). The service control unit 112 checks whether or not the terminal device has received the video (S351), and a billing process is executed if necessary when the video has been received (S352).

Here, the above-described exchange between the terminal device and the server may be executed between a single terminal device and a single server, or between a plurality of terminal devices used by users and/or a plurality of server devices cooperating via a network. For example, there can be an example in which the issuance of the request in S341, the checking of downloading in S347, and the reception of the video in S350 are executed by a single terminal device (e.g., which can be a wearable device or a mobile device). On the other hand, the issuance of the request in S341 may be executed by a server. In addition, when a user is wearing a plurality of wearable devices or mobile devices, the user can check downloading using a different terminal device from the terminal device that issued the request or can receive a video using another terminal device. For example, the thumbnail image may be simultaneously transmitted to a plurality of terminal devices in S346, and any one of the plurality of terminal devices, more specifically, a terminal device that acquired a response of the user, may execute checking of downloading in S347. In addition, at a time at which the download is checked in S347, the user may be able to select or designate a terminal device that will receive the video in S350. In such a case, the terminal device that will receive the video in S350 is not limited to a wearable device or a mobile device, and may be a terminal device that is located away from the user at that point of time (e.g., present in the user's house or the like).

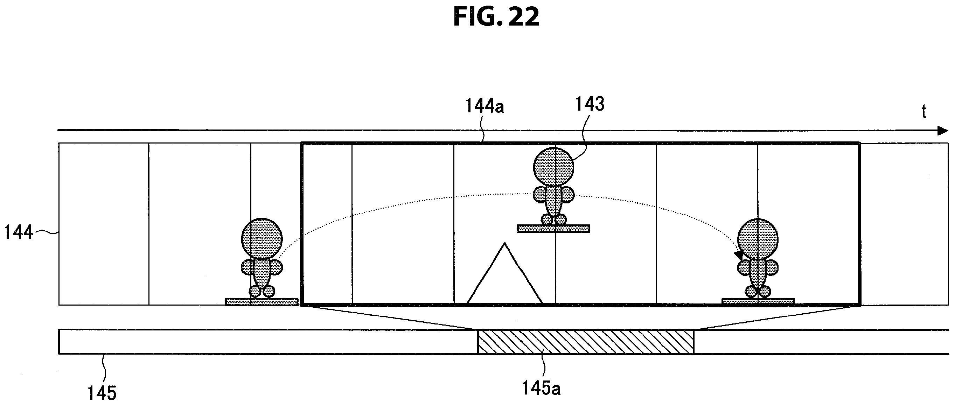

FIG. 22 is a diagram for describing a video processing in the process exemplified in FIG. 21. Note that, in the illustrated example, a detected action includes a jump. In S349 of the process shown in FIG. 21, the service control unit 112 executes cut-out processing on the section corresponding to the action section from the video data. At this time, the section 144a cut out of a video 144 can be a section enlarged a predetermined length or a predetermined ratio more than a section 145a detected as an action section (a jump section in the illustrated example) of the user 143 in an action detection result 145. In a case of a jump section, for example, it is desirable for a portion before a takeoff of the jump and a portion after landing to be included in a video. In such a case, the service control unit 112 causes the section 144a of the video to be enlarged more than the detected action section 145a.

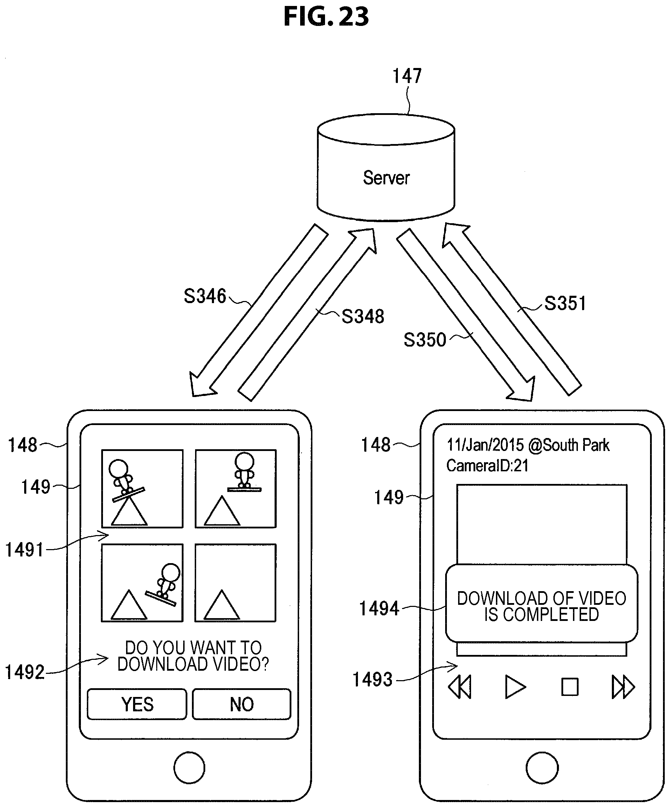

FIG. 23 is a diagram for describing a user interface for downloading a video in the process shown in FIG. 21. In FIG. 23, the processes of S436, S348, S350, and S351 shown FIG. 21 are indicated as communication between a server 147 (which can be the information processing device 100) and a terminal device 148. For example, in the terminal device 148 (which is a mobile device such as a tablet or a smartphone in the illustrated example) that receives a thumbnail image a server 147 in the process of S346, a display 149 is caused to display the thumbnail image 1491 and a dialogue 1492 for checking whether or not a download of the video is consented to. Here, the user decides, for example, whether to consent to the download through input of an operation in a touch panel provided on the display 149. When the download is consented to, a download request is transmitted from the terminal device 148 to the server 147 (S348).

When it is determined the user has consented in S348 as described above, a processed image is downloaded into the terminal device 148 from the server 147 (S350). At this time, the display 149 of the terminal device 148 may display a reproduction screen 1493 for the downloaded video and a message 1494 notifying the user that the download has completed. When the download is completed, a notification of completion of reception is transmitted from the terminal device 148 to the server 147 (S351), and the server 147 transitions to a billing process or the like thereafter.

(4-4. Fourth example)

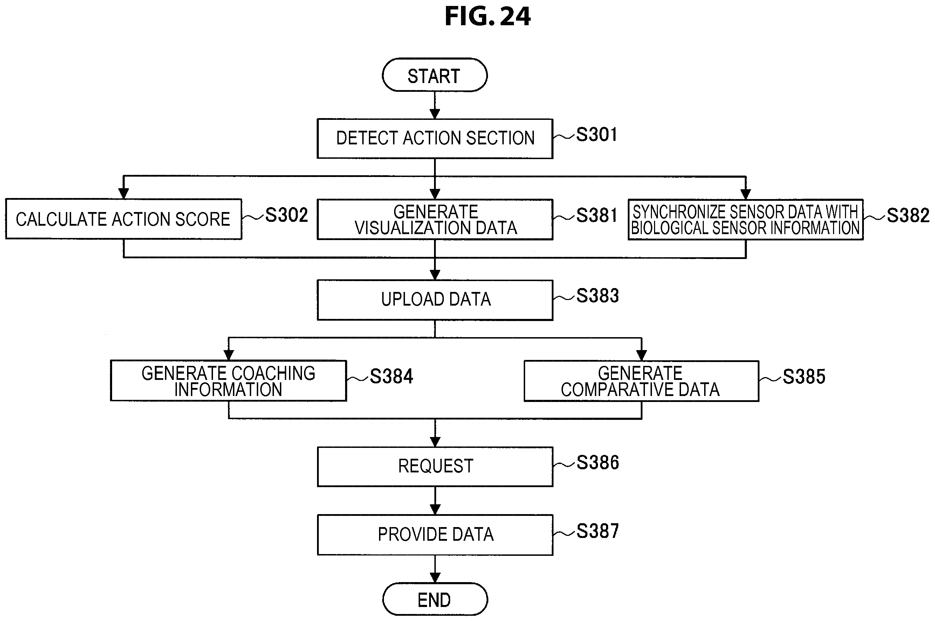

FIG. 24 is a flowchart showing a process of a fourth example of information generation included in an embodiment of the present disclosure. In the present example, information for supporting improvement of an action (e.g., a play in a sport) is provided on the basis of information of a detected action section.

In the illustrated example, first, a process of a detection of an action section is executed (S301) by the action detection unit 106 included in the sensor data analysis unit 104 as in the first to the third examples. Next, the scoring processing unit 109 included in the analysis result processing unit 107 may calculate an action score as in the first and second examples (S302). In addition, the analysis result processing unit 107 may generate data for visualizing various kinds of physical amounts of the action section or feature amounts extracted from the physical amounts (S381). The data generated here is visualized while, for example, statistics relating to the action are updated in real time, and thereby the data can be used as useful information for improving the action. Further, the analysis result processing unit 107 may synchronize sensor data used when detecting the action with data of a biological sensor separately mounted on the user for the action section (S382).

The above-described data generated by the analysis result processing unit 107 is, for example, uploaded from a sever realizing the function of the analysis result processing unit 107 to a server realizing the service control unit 112 (S383). Alternatively, the uploading in S383 may be performed from a terminal device realizing the function of the analysis result processing unit 107 to a server realizing the service control unit 112. When these servers or terminal device are the same, the uploading can be read as, for example, registration in an internal database.

The service control unit 112 that receives the upload of the generated information in S383 executes a generation of coaching information (S384), and/or a generation of comparative data (S385). The coaching information generated in S384 is extracted from a transition from past history data to the latest data of the user with regard to the uploaded information, for example, intensity of practice (calculated using a pulse rate and respiratory rate), the pulse rate, the respiratory rate, a degree of stability/instability of a motion, an action score, and the like. More specifically, the coaching information includes information for notifying the user of a time at which to take a break on the basis of a level of fatigue (estimated from an action history) and a level of concentration, estimating a good or a bad condition of the user, or notifying the user of a detection of a superior action in comparison to the past history data. In addition, the comparative data generated in S385 is data for quantitatively ascertaining points to be improved by, for example, comparing a newly detected action to past data of the user or data of another user. Such comparative data can be extracted by, for example, matching inclinations, directions, heights, levels of stability/instability of motions, head down level, impacts, speeds (vectors), accelerations (vectors), rotation vectors (quaternions), and the like of the user in a time series manner and eliciting their correlations and differences.

After the generation of the data in S384 and/or S385, a data request is issued (S386). The request may be transmitted, for example, from the server or the terminal device that has uploaded the information in S383 realizing the function of the analysis result processing unit 107 to a server realizing the service control unit 112. When these servers or terminal device are the same, the issuance of the request can be read as, for example, an internal issuance of a command for requesting data. In response to the request, the service control unit 112 provides data to the server or the terminal device that has issued the request (S387). The server may further transfer the provided data to a terminal device. The data provided to the terminal device is output by, for example, a user interface for presenting information to be displayed on a screen.

(Example of screen display)

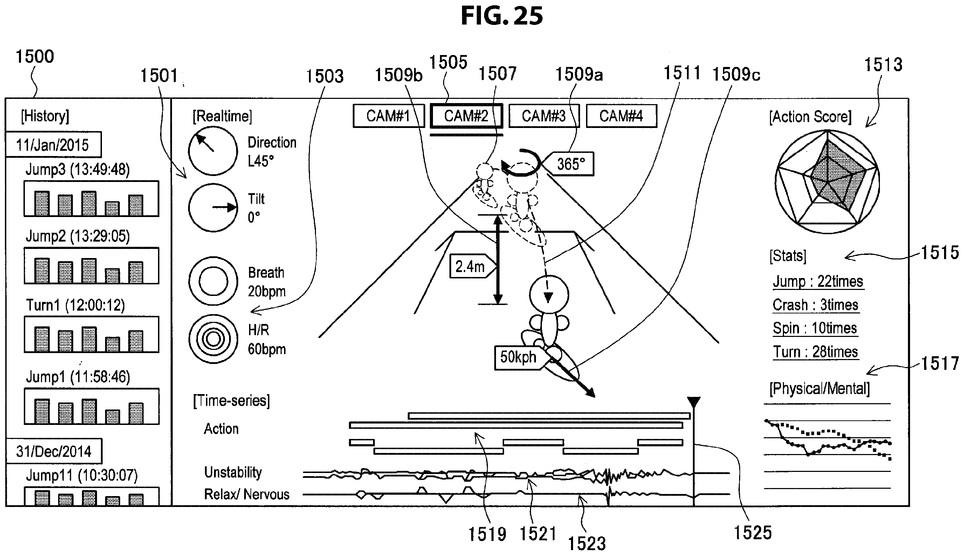

FIG. 25 is a diagram illustrating a screen display in the fourth example of information generation included in an embodiment of the present disclosure. Referring to FIG. 25, a screen 1500 displays a bearing-and-inclination 1501 of a user detected in real time, biological information (a respiratory rate and a pulse rate) 1503 of the user detected in real time, switch buttons 1505 for a plurality of viewpoints, an afterimage 1507 of the user in a past portion of an action section, a physical amount display 1509, a trace 1511 of the user in the action section, a radar chart 1513 of action scores, statistics 1515, a graph 1517 indicating a physical/psychological state, a time-series display of a detected action 1519, a time-series display of a level of instability of a motion 1521, a time-series display of a degree of relaxation/tension 1523, and a seekbar 1525 for referring to the time-series displays.

For example, the above-described bearing-and-inclination 1501, the physical amount display 1509, the radar chart 1513, and the statistics 1515 can be generated through, for example, the process of S381 shown in FIG. 24. In the illustrated example, the physical amount display 1509 includes a rotation angle display 1509a, a jump height display 1509b, and a speed display 1509c. These display elements visualize, for example, various physical amounts of the action section, feature amounts extracted from the physical amounts, and values calculated on the basis of feature amounts such as action scores. If, for example, the information is visualized while being updated in real time, it is easy for the user to ascertain points to be improved in his or her action.

In addition, the above-described biological information 1503, graph 1517, and time-series displays 1521 and 1523 can be generated through, for example, the process of S382 shown in FIG. 24. These display elements are displayed, for example, on the basis of a detection value of a biological sensor and an analysis result thereof in the action section. Note that a level of instability of a motion is extracted from a detection value of an inertial sensor including an acceleration sensor and an angular velocity sensor. Since biological information synchronized with the action section is fed back, it is easy to ascertain a psychological state or the like of the user when he or she performs an action, which leads to improvement in actions.

(4-5. Regarding user profile)

In an embodiment of the present disclosure as described above, a user profile can be created using a type of action detected in the past. For example, a profile of a "high level jumper" can be given to a user for which a high action score was calculated without problem for his or her action of jumping. In addition, a profile of a "low level jumper" can be given to a user who got a low action score for his or her jumping or whose toppling was detected during jumping.

For example, processes of information generation in the present embodiment can differ using the above-described profile. When information for supporting improvement of the user is provided in the example illustrated in, for example, FIGS. 24 and 25, the information may be reflected on the user profile. More specifically, when the user is highly skilled in jumping, visualized information regarding a jump in which toppling is rarely detected may be provided in more detail. The reason for such a provision is that, since it is a matter of course that highly-skilled users will succeed in jumping, it can be considered that a close analysis of a rare failure supports further improvement. Conversely, when the user is a beginning jumper, visualized information regarding a successful jump in which no toppling has been detected may be provided in more detail. The reason for such a provision is that, by reviewing a successful jump, there is a possibility for a beginning jumper getting a hint on succeeding in stable jumping.

(5. Hardware Configuration)

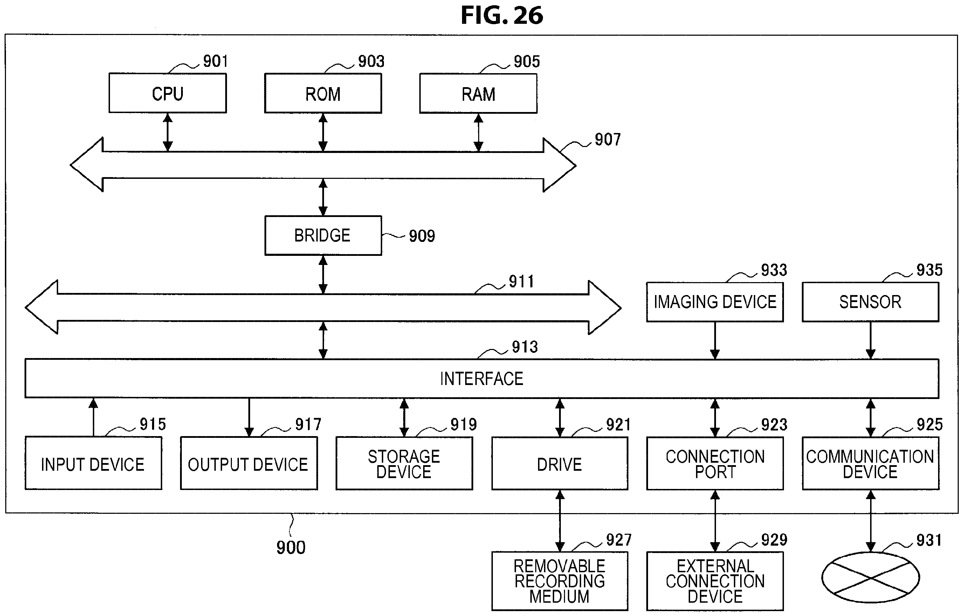

Next, with reference to FIG. 26, a hardware configuration of an information processing device according to an embodiment of the present disclosure is explained. FIG. 26 is a block diagram illustrating a hardware configuration example of an information processing device according to the embodiment of the present disclosure.

The information processing device 900 includes a central processing unit (CPU) 901, read only memory (ROM) 903, and random access memory (RAM) 905. In addition, the information processing device 900 may include a host bus 907, a bridge 909, an external bus 911, an interface 913, an input device 915, an output device 917, a storage device 919, a drive 921, a connection port 923, and a communication device 925. Moreover, the information processing device 900 may include an imaging device 933, and a sensor 935, as necessary. The information processing device 900 may include a processing circuit such as a digital signal processor (DSP), an application-specific integrated circuit (ASIC), or a field-programmable gate array (FPGA), alternatively or in addition to the CPU 901.

The CPU 901 serves as an arithmetic processing apparatus and a control apparatus, and controls the overall operation or a part of the operation of the information processing device 900 according to various programs recorded in the ROM 903, the RAM 905, the storage device 919, or a removable recording medium 927. The ROM 903 stores programs, operation parameters, and the like used by the CPU 901. The RAM 905 transiently stores programs used when the CPU 901 is executed, and various parameters that change as appropriate when executing such programs. The CPU 901, the ROM 903, and the RAM 905 are connected with each other via the host bus 907 configured from an internal bus such as a CPU bus or the like. The host bus 907 is connected to the external bus 911 such as a Peripheral Component Interconnect/Interface (PCI) bus via the bridge 909.

The input device 915 is a device operated by a user such as a mouse, a keyboard, a touch panel, a button, a switch, and a lever. The input device 915 may be a remote control device that uses, for example, infrared radiation and another type of radiowave. Alternatively, the input device 915 may be an external connection device 929 such as a mobile phone that corresponds to an operation of the information processing device 900. The input device 915 includes an input control circuit that generates input signals on the basis of information which is input by a user to output the generated input signals to the CPU 901. A user inputs various types of data to the information processing device 900 and instructs the information processing device 900 to perform a processing operation by operating the input device 915.

The output device 917 includes an apparatus that can report acquired information to a user visually, audibly, or haptically. The output device 917 may be, for example, a display device such as a liquid crystal display (LCD) or an organic electro-luminescence (EL) display, an audio output device such as a speaker or a headphone, or a vibrator. The output device 917 outputs a result obtained through a process performed by the information processing device 900, in the form of video such as text and an image, sounds such as voice and audio sounds, or vibration.

The storage device 919 is an apparatus for data storage that is an example of a storage unit of the information processing device 900. The storage device 919 includes, for example, a magnetic storage device such as a hard disk drive (HDD), a semiconductor storage device, an optical storage device, or a magneto-optical storage device. The storage device 919 stores therein the programs and various data executed by the CPU 901, various data acquired from an outside, and the like.

The drive 921 is a reader/writer for the removable recording medium 927 such as a magnetic disk, an optical disc, a magneto-optical disk, and a semiconductor memory, and built in or externally attached to the information processing device 900. The drive 921 reads out information recorded on the mounted removable recording medium 927, and outputs the information to the RAM 905. Further, the drive 921 writes the record into the mounted removable recording medium 927.

The connection port 923 is a port used to connect devices to the information processing device 900. The connection port 923 may include a Universal Serial Bus (USB) port, an IEEE1394 port, and a Small Computer System Interface (SCSI) port. Further, the connection port 923 may further include an RS-232C port, an optical audio terminal, a High-Definition Multimedia Interface (HDMI) (registered trademark) port, and so on. The connection of the external connection device 929 to the connection port 923 makes it possible to exchange various data between the information processing device 900 and the external connection device 929.

The communication device 925 is a communication interface including, for example, a communication device for connection to a communication network 931. The communication device 925 may be, for example, a communication card for a wired or wireless local area network (LAN), Bluetooth (registered trademark), a near field communication (NFC), or a wireless USB (WUSB). Further, the communication device 925 may also be, for example, a router for optical communication, a router for asymmetric digital subscriber line (ADSL), or a modem for various types of communication. For example, the communication device 925 transmits and receives signals in the Internet or transits signals to and receives signals from another communication device by using a predetermined protocol such as TCP/IP. In addition, the communication network 931 to which the communication device 925 connects is a network established through wired or wireless connection. The communication network 931 may include, for example, the Internet, a home LAN, infrared communication, radio communication, or satellite communication.

The imaging device 933 is an apparatus that captures an image of a real space by using an image sensor such as a charge coupled device (CCD) and a complementary metal oxide semiconductor (CMOS), and various members such as a lens for controlling image formation of a subject image onto the image sensor, and generates the captured image. The imaging device 933 may capture a still image or a moving image.

The sensor 935 is various sensors such as an acceleration sensor, an angular velocity sensor, a geomagnetic sensor, an illuminance sensor, a temperature sensor, a barometric sensor, a pressure sensor, a distance sensor, and a sound sensor (microphone). The sensor 935 acquires information regarding a state of the information processing device 900 such as a posture of a housing of the information processing device 900, and information regarding an environment surrounding the information processing device 900 such as luminous intensity and noise around the information processing device 900. The sensor 935 may include a global navigation satellite system (GNSS) receiver that receives GNSS signals to measure latitude, longitude, and altitude of the apparatus.

The example of the hardware configuration of the information processing device 900 has been described. Each of the structural elements described above may be configured by using a general purpose component or may be configured by hardware specialized for the function of each of the structural elements. The configuration may be changed as necessary in accordance with the state of the art at the time of working of the present disclosure.

(6. Supplement)

The present disclosure may include, for example, the above-described information processing device, the above-described system, the information processing method executed by the information processing device or the system, a program for causing the information processing device to exhibits its function, and a non-transitory physical medium having the program stored therein.

(4. Examples of content control)

Several examples of content control included in an embodiment of the present disclosure will be described below. In the several examples, the service control unit 112 of the information processing device 100 controls, for example, a provision of visual or acoustic content provided in parallel with progress of an action. Such content can include, for example, a visual effect, a sound effect, and the like provided to a user who performs an action and another user who observes the action in real time using a head mounted display (HMD) or the like. Alternatively, the content may include a visual effect, a sound effect, and the like reproduced after the fact with a video of an action. In addition, in several other examples, the content may include a video of an action itself.

(7-1. Control using time-series scores)

In the present embodiment, the service control unit 112 of the information processing device 100 calculates time-series scores for a section including a series of actions of a user detected by the action detection unit 106 and executes content control using the scores as a first example.

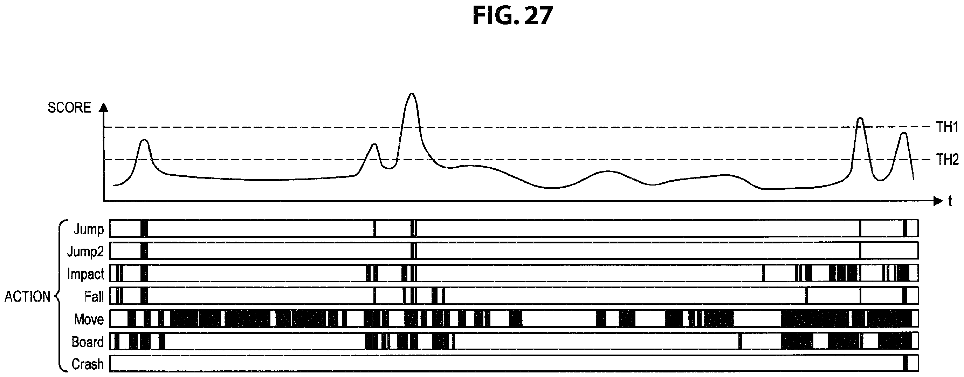

FIG. 27 is a diagram for describing a calculation of time-series scores for content control in an embodiment of the present disclosure. In the illustrated example, jumps (e.g., detected using the detection methods of the two above-described examples), impacts, (free) falls, movements, on-board states, and topplings (crashes) are detected as actions of a snowboarding user. The service control unit 112, for example, weights action scores of each of actions detected by the action detection unit 106 included in the sensor data analysis unit 104 and sums the results when necessary, and thereby calculates time-series scores for content control as in the illustrated example. A time-series score S(t1) at a time t1 is calculated using the following formula 1 on the basis of, for example, a score of each action $S_{Action}(t1)$ and a weight $W_{Action}$ set for each of the actions (Action indicates the name of an action).

[Math. 1]

$$S(t1) = W_{Jump} \cdot S_{Jump}(t1) + W_{Jump2} \cdot S_{Jump2}(t1) + \cdots + W_{Crash} \cdot S_{Crash}(t1) \quad \cdot \cdot \cdot (\text{Formula 1})$$

Here, the score of each action $S_{Action}(t1)$ may simply indicate whether or not the action occurred at the time t1 (in this case, $S_{Action}(t1)$ can be 0/1), the time t1, or a score calculated by the scoring processing unit 109 for an action section that is included in the time t1. When a time-series score that gently changes as in the illustrated example is calculated, for example, the scores of respective actions $S_{Action}(t1)$ may be smoothed and summed. In addition, the weight $W_{Action}$ of each of the actions can be set in accordance with, for example, levels of importance of the actions when snowboarding.

The service control unit 112 may set, for example, one or a plurality of threshold values (TH1 and TH2) for the calculated time-series scores and execute content control with reference to whether or not each of the time-series score exceeds the threshold value. For example, the service control unit 112 may cause any content to be continuously generated or output for a section whose time-series score exceeds the threshold value. Alternatively, the service control unit 112 may cause any content to be generated or output for at least a first part and a final part of a section whose time-series score exceeds the threshold value.

As a more specific use example, the service control unit 112 may extract a highlight of an action video on the basis of a time-series score. The action video is, for example, a video obtained by capturing a user performing an action and acquired in parallel with sensor data (a sensor device that acquires the sensor data and an imaging device that acquires the action video can be different devices). In the illustrated example, the service control unit 112 may extract, for example, a section whose time-series score exceeds the threshold value (TH1 or TH2) from an action video continuously captured while sliding during snowboarding as a highlight video. This section is defined using, for example, a timing at which the time-series score increases and exceeds the threshold value when an action has occurred and a timing at which the time-series score decreases thereafter and falls under the threshold value. Which one of the threshold value TH1 and the threshold value TH2 will be used in the extraction determination is determined in accordance with, for example, a length of the highlight video designated in advance. In the case of the illustrated example, a shorter highlight video is extracted if the threshold value TH1 is used in the determination and a longer highlight video is extracted if the threshold value TH2 is used in the determination.

In addition, as another example, the service control unit 112 may control a reproduction of an action video on the basis of a time-series score. More specifically, when an action video of which a highlight has not been extracted unlike in the above-described example is to be reproduced, the service control unit 112 may reproduce the action video by skipping a section whose time-series score is lower than the threshold value (TH1 or TH2) automatically or in accordance with a seek operation of the user. Similarly to the above-described example, which one of the threshold value TH1 and the threshold value TH2 will be used in the skipping determination is determined in accordance with, for example, a reproduction time designated in advance. In the case of the illustrated example, the action video is reproduced for a shorter period of time if the threshold value TH1 is used in the determination and the action video is reproduced for a longer period of time if the threshold value TH2 is used in the determination.

In addition, for example, the service control unit 112 may execute content control in accordance with a calculated time-series score value. For example, the service control unit 112 may control whether or not an effect added to a reproduction of an action video or a score itself in a certain section (which can also be defined in accordance with a time-series score as in the above-described example) is to be displayed in accordance with a time-series score value (e.g., a mean, a maximum value, or the like) for the section. In addition, for example, the service control unit 112 may select music to be reproduced along with the action video and a volume thereof in accordance with the time-series score value. More specifically, the service control unit 112 may reproduce cheerful music at a high volume for a section having a high time-series score, and may reproduce quiet music at a low volume for a section having a low time-series score.

Note that the calculation of a time-series score for the above-described content control is not limited to actions of snowboarding users, and can be applied to actions performed in various scenes in other sports, daily lives, and the like. For example, the time-series score may be calculated by summing scores of a plurality of actions that occurred in scenes (the value of 0/1 indicates the occurrence or calculated scores), or a score of a single action may be used as a time-series score without change. In addition, a score of an element other than an action detected on the basis of sensor data, more specifically, a score based on an input of an operation of a user or a score based on a detection of position information or check-in information (defined by, for example, a check-in through an explicit operation of a user using NFC or the like, a geo-fence using position information, a radio wave reception range of a wireless LAN or the like, a radio station cluster disclosed in WO 2014/125689, or the like) may be used in the calculation of a time-series score, along with an action score. In addition, in such a case, the service control unit 112 may vary a control of a provision of content with respect to an action that occurs in a sport and an action that does not occur in the sport.

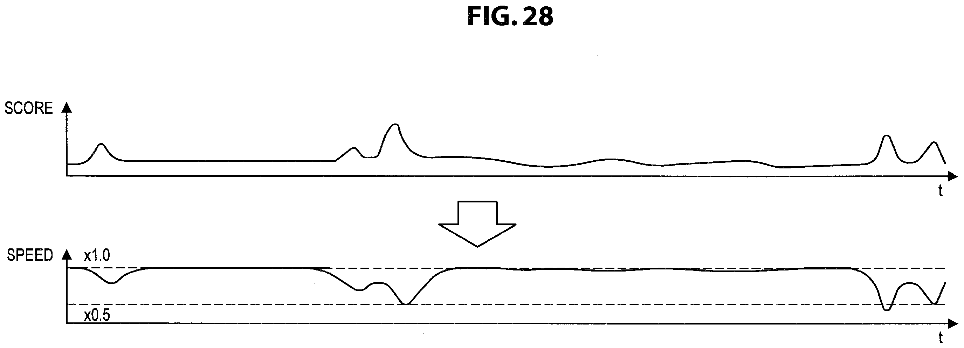

FIG. 28 is a diagram for describing an example of content control using a time-series score in an embodiment of the present disclosure. In the illustrated example, a reproduction speed of an action image is continuously controlled on the basis of time-series scores. More specifically, the service control unit 112 may multiply the time-series score $S(t1)$ at the certain time t1 by a predetermined coefficient $\alpha$ to decide a reproduction speed $Sp(t1)$ at the time t1. That is,

$$Sp(t1) = \alpha \cdot S(t1).$$

In the illustrated example, a negative coefficient $\alpha$ is set such that the reproduction speed is a normal speed (1.0 time) when the time-series score has a minimum value and the reproduction speed is half the normal speed (0.5 times) when the time-series score has a maximum value. Accordingly, the action video can be automatically reproduced slowly at a part having a high time-series score. As another example, if the reproduction speed is the normal speed (1.0 time) when the time-series score has the maximum value and the reproduction speed is set to further increase (>1.0 time) as the time-series score decreases, the action video can be automatically fast-forwarded at a part having a low time-series score.

(7-2. Control using timing defined for action)

As a second example, the service control unit 112 of the information processing device 100 specifies at least two timings defined for an action of a user detected by the action detection unit 106 and executes content control in accordance with the timings in the present embodiment.

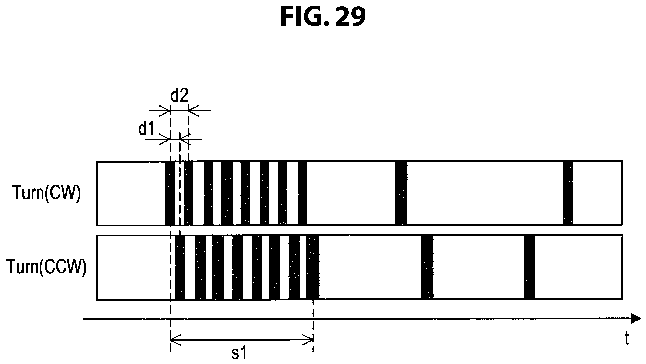

FIG. 29 is a diagram for describing an example of timings defined for an action in an embodiment of the present disclosure. In the illustrated example, the action detection unit 106 executes, for example, the detection process as described above with reference to FIG. 10 to detect a turn of a user. In the present example, the action detection unit 106 identifies a clockwise turn (CW) and a counterclockwise turn (CCW) in accordance with a rotation direction.

In the diagram, detection timings of the two types of turns are illustrated in a time series manner. For example, when an action of a user sliding during snowboarding is detected, a section in which clockwise turns and counterclockwise turns regularly and consecutively occur at substantially uniform time intervals can exist as in the illustrated example. In this case, the service control unit 112 can define, for example, a section s1 that includes a first timing and a final timing of consecutive turns, an interval d1 between timings of turns which have different rotation directions, an interval d2 between timings of turns which have the same rotation directions, and the like on the basis of a series of timings defined for the turns that have consecutively occurred, and thus can execute content control on the basis of the results.

For example, the service control unit 112 may select music with a tempo corresponding to the interval d1 or the interval d2 and reproduce the music along with an action video in a section including at least a part of the section s1. At this time, the service control unit 112 reproduces the music in the section including at least the section s1. More specifically, for example, the service control unit 112 may start reproducing the music from a beginning point of the section s1 (a timing of a first turn) or earlier. Alternatively, the service control unit 112 may reproduce such music in real time during the sliding of the user. In this case, the service control unit 112 selects the music and starts reproducing the music at a time point at which the several intervals d1 and intervals d2 are consecutively detected in the beginning point of the section s1. The service control unit 112 may finish reproducing the music at an ending point of the section s1 (a timing of a last turn) using a fade-out or the like, and may continue to reproduce the music until a subsequent set of consecutive turns are detected, for example, even after the end of the section s1.

Here, music with a tempo corresponding to the interval d1 or the interval d2 can be music played at, for example, a reciprocal of the interval d1 or the interval d2, i.e., beats per minute (BPM), close to a turn appearance frequency. At this time, by finely adjusting the music with the tempo at the BPM close to the turn appearance frequency, the music may match the turn appearance frequency.

In addition, for example, the service control unit 112 may generate a visual effect (e.g., an animation, an avatar, or the like) or a sound effect that develops (e.g., is repeated) at timings corresponding to the intervals d1 or the intervals d2 and cause the effects to be reproduced along with the action video in the section including at least a part of the section s1. Similarly to the above-described example of music, the service control unit 112 may provide the visual effect or sound effect using, for example, an HMD being worn by the user or the like.

Note that the above-described content control using timings defined for actions is not limited to the case of turns that consecutively occur at the time of sliding during snowboarding, and can also be applied to, for example, consecutive jumps and the like. In addition, the above-described control can be likewise applied to a case in which any type of action periodically occurs in other sports, daily lives, and the like. In such a case, the service control unit 112 may vary control of a provision of content with respect to an action that occurs in a sport and an action that does not occur in the sport. In addition, in this case, when music is selected as in the above-described example, for example, high-order behavior information of a user, which is estimated on the basis of a detected action and position information, a context before and after the occurrence of the action, or the like, may be used. For example, if such high-order behavior information indicating whether walking, which is performed as a periodically occurring action when music is reproduced, is for moving forward, for strolling, or for shopping can be used, music exactly appropriate for a situation of the user can be selected.

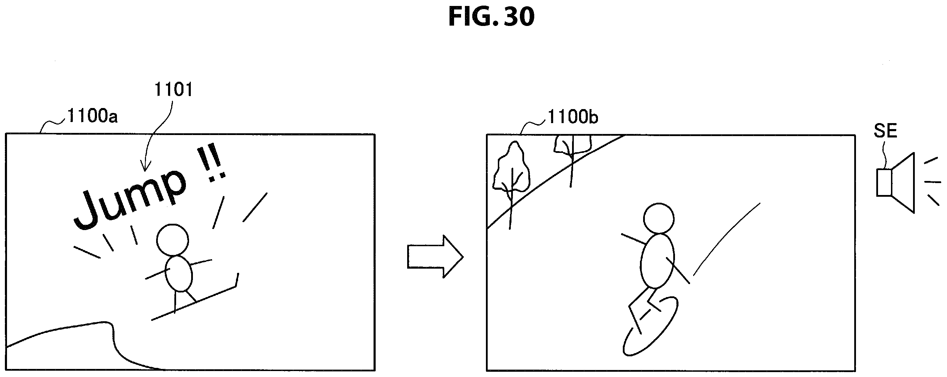

FIG. 30 is a diagram illustrating a first example of effects provided at timings defined for actions in an embodiment of the present disclosure. In the illustrated example, a visual effect and a sound effect are provided in accordance with timings of a takeoff and landing of a jump on the screens 1100 on which an action video is reproduced. More specifically, in the illustrated example, a visual effect 1101 using the text "Jump!!" is displayed on the screen 1100a during the takeoff of the jump. In addition, a sound effect SE is output on the screen 1100b during the landing of the jump.

Here, an effect to be provided may be changed, for example, in accordance with an action score calculated by the scoring processing unit 109 included in the analysis result processing unit 107. Although both the visual effect 1101 and the sound effect SE are provided in the example illustrated in FIG. 30, for example, only the visual effect 1101 may be provided when the action score is low. Further, when the action score is low, a size of the visual effect 1101 may be small or the effect may be plain. Examples of types of action and control of types of effect in accordance with action scores are shown in Table 1 below.

[Table 1]

|  | High action score | Low action score |
|---|---|---|
| Jump | Image (large Jump!!) Sound effect | Image (small Jump) |
| Turn | Sound effect (loud) | Sound effect (quiet) |
| Toppling | Negative-positive reversal | Sound effect |

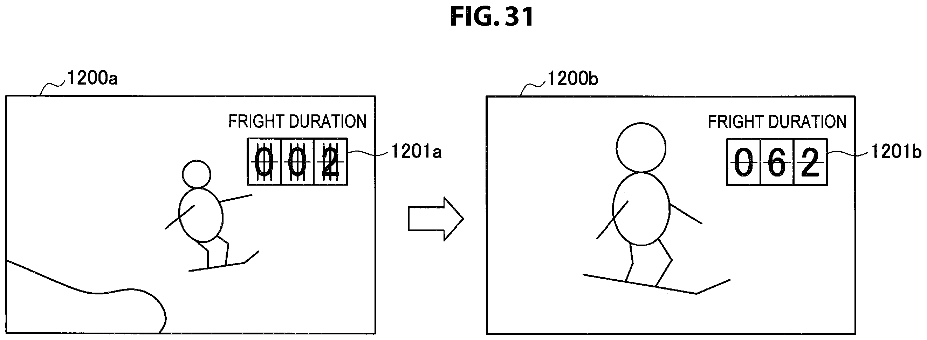

FIG. 31 is a diagram illustrating a second example of effects provided at timings defined for actions in an embodiment of the present disclosure. In the illustrated example, animations 1201 that are a type of visual effect are displayed at timings of a takeoff and landing of a jump on the screens 1200 on which an action video is reproduced. More specifically, in the illustrated example, an animation 1201a representing a counter that indicates flight duration and rotates is displayed on the screen 1200a during the takeoff of the jump. In addition, an animation 1201b in which the counter stops and the flight duration is confirmed is displayed on the screen 1200b during the landing of the jump. In more detail, the counter in the animation 1201a may count up a time during which the user is in a flight state after the takeoff and stop counting at a time point of the landing.

The service control unit 112 may change an operation of a series of visual effects and sound effects on the basis of timings defined for actions as described above. More specifically, when two timings (a first timing and a second timing) of a takeoff and landing of a jump as in the above-described example are defined, the service control unit 112 may provide a visual effect or a sound effect which changes an operation thereof at least at one timing.

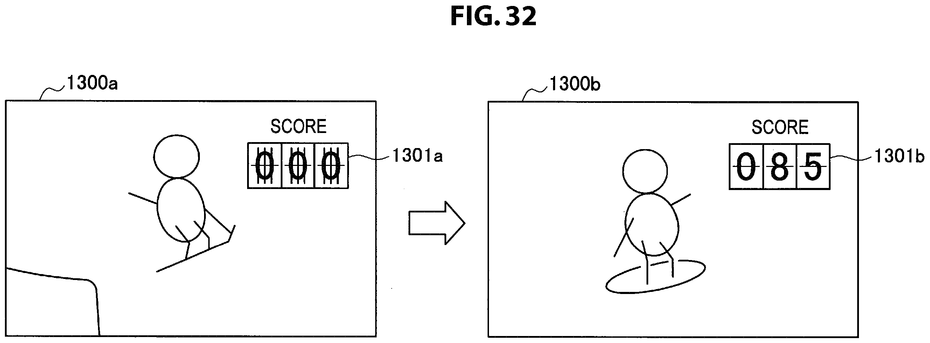

FIG. 32 is a diagram illustrating a third example of effects provided at timings defined for actions in an embodiment of the present disclosure. In the illustrated example, animations 1301 are displayed at timings of a takeoff and landing of a jump on screens 1300 on which an action image is reproduced, as in the second example. More specifically, in the illustrated example, an animation 1301a representing a counter that indicates an action score of a jump and rotates is displayed on a screen 1300a during the takeoff of the jump. In addition, an animation 1301b in which the counter has stopped and the action score is confirmed is displayed on a screen 1300b during the landing of the jump. In more detail, the counter of the animation 1301a may show randomly changing scores while the user is in the flight state after the takeoff and stop at a time point of the landing. Alternatively, in order to reproduce a state of grading after the jump is executed, the rotating counter may be displayed in the animation 1301a immediately after the landing of the jump and the animation 1301b in which the action score is confirmed may be displayed after a predetermined period of time elapses. In this case, for example, the animations 1301 may also be displayed in real time using an HMD worn by the user or the like.

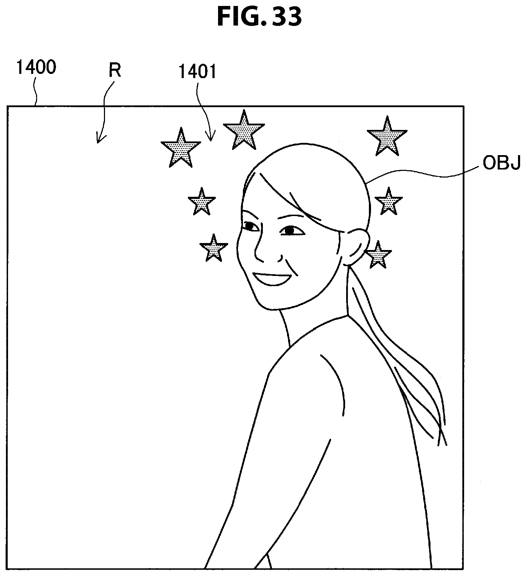

FIG. 33 is a diagram illustrating a fourth example of an effect provided at timings defined for actions in an embodiment of the present disclosure. In the illustrated example, in an image 1400 displayed to be superimposed on the real space image R using an HMD or the like, a visual effect 1401 of stars is displayed around the face of an object (OBJ), which is a person in the real space, at a time at which the object (OBJ) looks back (head shake).

The display of the visual effect 1401 is possible by, for example, detecting an action of head shake of the object (OBJ) on the basis of sensor data provided by an acceleration sensor, an angular velocity sensor, and the like mounted on the object (OBJ) and displaying the visual effect 1401 in the image 1400 at a timing at which the head shake is detected. A position of the face of the object (OBJ) can be specified using, for example, a known face detection technology. An animation or a sound effect may be output in a similar manner as the visual effect 1401.

(7-3. Regarding profile of user)

A user profile can be created depending on types of action detected in the past. For example, a profile of a "high level jumper" is given to a user for whom a high action score has been stably calculated for his or her jumping action. In addition, a profile of a "beginning jumper" is given to a user for whom a low action score has been calculated for his or her jump and many times of toppling have been detected during jumps.

Processes of content control in the present embodiment may differ using, for example, the above-described profiles. For example, when a highlight video is generated using the time-series score calculated in the example illustrated in FIG. 27, a section to be extracted with priority may be decided on the basis of a profile of a user. More specifically, when the user is a high level jumper, a section of a jump in which toppling is rarely detected may be extracted with priority. The reason for such an extraction is that, since it is a matter of course for high level jumpers to succeed in jumping, it is considered that closely analyzing a rare failure supports further improvement. In addition, high level jumpers are normally considered to view and enjoy videos of their successful jumps, and thus a controlled effect, for example, a time-lapse effect may be added.

On the other hand, when a user is a beginning jumper, a section of a successful jump in which toppling has not been detected may be extracted with priority. The reason for this is that, by reviewing the successful jump, there is a possibility for the beginning jumper to get a hint on stably succeeding in jumping. Alternatively, in order for the beginning jumper to easily compare successful jumps and failed jumps, videos of jumps that are extracted to be continuously reproduced may be arranged. In addition, beginning jumpers are normally considered to be pleased with the fact that they have succeeded in jumping, and thus a fancy effect, for example, an animation, an avatar, or a sound effect may be added.

(8. Hardware Configuration)

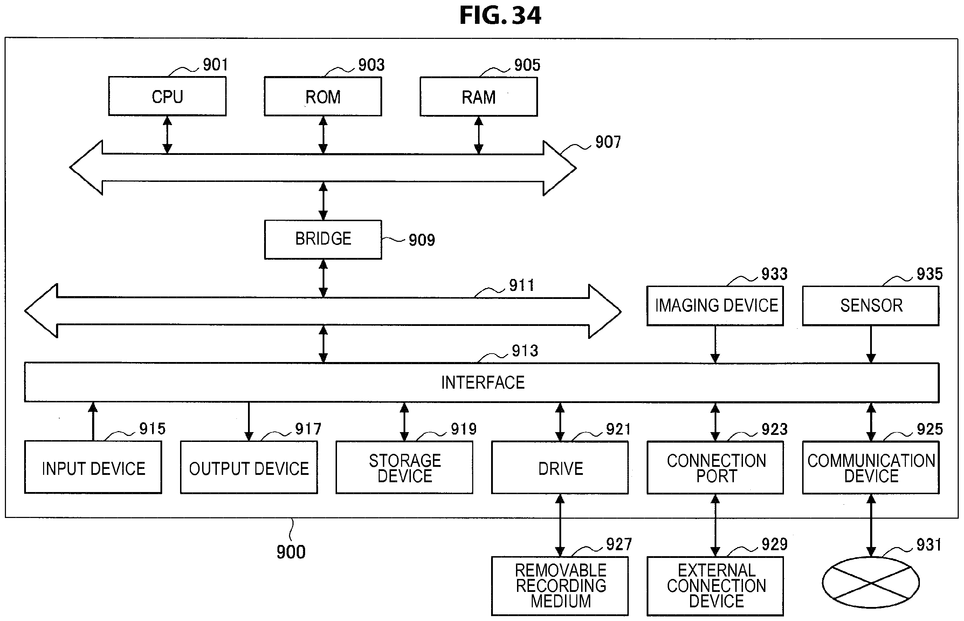

Next, with reference to FIG. 34, a hardware configuration of an information processing device according to an embodiment of the present disclosure is explained. FIG. 34 is a block diagram illustrating a hardware configuration example of an information processing device according to the embodiment of the present disclosure.

The information processing device 900 includes a central processing unit (CPU) 901, read only memory (ROM) 903, and random access memory (RAM) 905. In addition, the information processing device 900 may include a host bus 907, a bridge 909, an external bus 911, an interface 913, an input device 915, an output device 917, a storage device 919, a drive 921, a connection port 923, and a communication device 925. Moreover, the information processing device 900 may include an imaging device 933, and a sensor 935, as necessary. The information processing device 900 may include a processing circuit such as a digital signal processor (DSP), an application-specific integrated circuit (ASIC), or a field-programmable gate array (FPGA), alternatively or in addition to the CPU 901.

The CPU 901 serves as an arithmetic processing apparatus and a control apparatus, and controls the overall operation or a part of the operation of the information processing device 900 according to various programs recorded in the ROM 903, the RAM 905, the storage device 919, or a removable recording medium 927. The ROM 903 stores programs, operation parameters, and the like used by the CPU 901. The RAM 905 transiently stores programs used when the CPU 901 is executed, and various parameters that change as appropriate when executing such programs. The CPU 901, the ROM 903, and the RAM 905 are connected with each other via the host bus 907 configured from an internal bus such as a CPU bus or the like. The host bus 907 is connected to the external bus 911 such as a Peripheral Component Interconnect/Interface (PCI) bus via the bridge 909.

The input device 915 is a device operated by a user such as a mouse, a keyboard, a touch panel, a button, a switch, and a lever. The input device 915 may be a remote control device that uses, for example, infrared radiation and another type of radiowave. Alternatively, the input device 915 may be an external connection device 929 such as a mobile phone that corresponds to an operation of the information processing device 900. The input device 915 includes an input control circuit that generates input signals on the basis of information which is input by a user to output the generated input signals to the CPU 901. A user inputs various types of data to the information processing device 900 and instructs the information processing device 900 to perform a processing operation by operating the input device 915.

The output device 917 includes an apparatus that can report acquired information to a user visually, audibly, or haptically. The output device 917 may be, for example, a display device such as a liquid crystal display (LCD) or an organic electro-luminescence (EL) display, an audio output device such as a speaker or a headphone, or a vibrator. The output device 917 outputs a result obtained through a process performed by the information processing device 900, in the form of video such as text and an image, sounds such as voice and audio sounds, or vibration.

The storage device 919 is an apparatus for data storage that is an example of a storage unit of the information processing device 900. The storage device 919 includes, for example, a magnetic storage device such as a hard disk drive (HDD), a semiconductor storage device, an optical storage device, or a magneto-optical storage device. The storage device 919 stores therein the programs and various data executed by the CPU 901, various data acquired from an outside, and the like.

The drive 921 is a reader/writer for the removable recording medium 927 such as a magnetic disk, an optical disc, a magneto-optical disk, and a semiconductor memory, and built in or externally attached to the information processing device 900. The drive 921 reads out information recorded on the mounted removable recording medium 927, and outputs the information to the RAM 905. In addition, the drive 921 writes the record into the mounted removable recording medium 927.

The connection port 923 is a port used to connect devices to the information processing device 900. The connection port 923 may include a Universal Serial Bus (USB) port, an IEEE1394 port, and a Small Computer System Interface (SCSI) port. In addition, the connection port 923 may further include an RS-232C port, an optical audio terminal, a High-Definition Multimedia Interface (HDMI) (registered trademark) port, and so on. The connection of the external connection device 929 to the connection port 923 makes it possible to exchange various data between the information processing device 900 and the external connection device 929.

The communication device 925 is a communication interface including, for example, a communication device for connection to a communication network 931. The communication device 925 may be, for example, a communication card for a wired or wireless local area network (LAN), Bluetooth (registered trademark), a near field communication (NFC), or a wireless USB (WUSB). In addition, the communication device 925 may also be, for example, a router for optical communication, a router for asymmetric digital subscriber line (ADSL), or a modem for various types of communication. For example, the communication device 925 transmits and receives signals in the Internet or transits signals to and receives signals from another communication device by using a predetermined protocol such as TCP/IP. Further, the communication network 931 to which the communication device 925 connects is a network established through wired or wireless connection. The communication network 931 may include, for example, the Internet, a home LAN, infrared communication, radio communication, or satellite communication.

The imaging device 933 is an apparatus that captures an image of a real space by using an image sensor such as a charge coupled device (CCD) and a complementary metal oxide semiconductor (CMOS), and various members such as a lens for controlling image formation of a subject image onto the image sensor, and generates the captured image. The imaging device 933 may capture a still image or a moving image.

The sensor 935 is various sensors such as an acceleration sensor, an angular velocity sensor, a geomagnetic sensor, an illuminance sensor, a temperature sensor, a barometric sensor, a pressure sensor, a distance sensor, and a sound sensor (microphone). The sensor 935 acquires information regarding a state of the information processing device 900 such as a posture of a housing of the information processing device 900, and information regarding an environment surrounding the information processing device 900 such as luminous intensity and noise around the information processing device 900. Further, the sensor 935 may include a global navigation satellite system (GNSS) receiver that receives GNSS signals to measure latitude, longitude, and altitude of the apparatus.

The example of the hardware configuration of the information processing device 900 has been described. Each of the structural elements described above may be configured by using a general purpose component or may be configured by hardware specialized for the function of each of the structural elements. The configuration may be changed as necessary in accordance with the state of the art at the time of working of the present disclosure.


(9. Supplement)


The present disclosure may include, for example, the above-described information processing device, the above-described system, the information processing method executed by the information processing device or the system, a program for causing the information processing device to exhibits its function, and a non-transitory physical medium having the program stored therein.


Reference Signs List

100   information processing device
101   transmission unit
102   reception unit
103   sensor device control unit
104   sensor data analysis unit
105   feature amount extraction unit
106   action detection unit
107   analysis result processing unit
108   clustering processing unit
109   scoring processing unit
112   service control unit


**Claims**

**1.** An information processing device (100) comprising:

a sensor data acquisition unit configured to acquire sensor data provided by a sensor worn by a user or mounted on a piece of equipment used by the user;

a sensor data analysis unit configured to detect a turn of the user on the basis of the sensor data, wherein detecting the turn includes detecting a rotation in an action of the user, detecting a non-turning rotation as noise with respect to the turn to be detected included in the sensor data, determining whether a non-turning rotation has been detected, wherein if a non-turning rotation is detected as the noise, detect the turn from the rotation from which the noise has been removed, and if no non-turning rotation has been detected, detecting the turn of the user based on the rotation; and

an information generation unit configured to generate information regarding the turn;

wherein the sensor includes a sensor mounted on a head on the user or a piece of equipment mounted on the head of the user,

wherein the non-turning rotation includes a rotation occurring through a head shake of the user, and

wherein the sensor data analysis unit is configured to detect the head shake of the user when clockwise and counterclockwise rotations are consecutively performed at a time interval that is smaller than or equal to a threshold value.

2. The information processing device (100) according to claim 1,

wherein the sensor includes an acceleration sensor and an angular velocity sensor, and

the action detection unit detects the action on a basis of a feature amount extracted from the sensor data, or, further comprising:

a scoring processing unit (109) configured to calculate a score for evaluating the turn,

wherein the information generation unit generates information regarding the turn on a basis of the score.

3. The information processing device (100) according to claim 2, wherein the information generation unit generates information including a skill level of the turn of the user estimated on a basis of the score.

4. The information processing device (100) according to claim 3, wherein the information generation unit generates information including a ranking that relates to a skill of the turn of the user, which is decided on a basis of the skill level, and/or, wherein the information generation unit generates information including an evaluation of each facility at which the turn is executed, which is decided on a basis of the skill level.

5. The information processing device (100) according to claim 3,

wherein the user includes a first user and a second user, and

the information generation unit generates information regarding the turn by comparing the first user and the second user with respect to the skill levels.

6. The information processing device (100) according to claim 5, wherein the information generation unit generates information for notifying the first user of presence of the second user when the skill level of the first user is close to the skill level of the second user, in particular, wherein the information generation unit generates the information for notifying the first user of the presence of the second user when positions of the first user and the second user are in proximity.

7. The information processing device (100) according to claim 2, wherein the scoring processing unit evaluates statistics of a duration, an angular displacement, an angular velocity, or an angular acceleration of the turn.

8. The information processing device (100) according to claim 1,

wherein the action includes the turn and a jump or toppling, and

the information generation unit generates information regarding the turn and the jump or the toppling.

9. The information processing device (100) according to claim 8, further comprising:

a scoring processing unit (109) configured to calculate a score for evaluating the jump,

wherein the scoring processing unit (109) evaluates a duration of the jump, an angular displacement of jumps, degrees of free fall of the jumps, and an impact at a time of a takeoff or landing of the jump, or, further comprising:

a scoring processing unit (109) configured to calculate a turn score for evaluating the turn and a jump score for evaluating the jump,

wherein the information generation unit generates information including a skill level of the user estimated on a basis of the turn score and the jump score.

10. The information processing device (100) according to claim 1, wherein the information generation unit generates information for notifying the user of presence of a video capturing the turn, and/or, wherein the information generation unit generates information for visualizing an amount of the turn in a time series manner.

11. An information processing method comprising:

acquiring sensor data provided by a sensor worn by a user or mounted on a piece of equipment used by the user;

detecting, by a processor, an action of the user on a basis of the sensor data, the action including a turn wherein processor further detects a turn of the user on the basis of the sensor data, wherein detecting the turn includes detecting a rotation in an action of the user, detecting a non-turning rotation as noise with respect to the turn to be detected included in the sensor data, determining whether a non-turning rotation has been detected, wherein if a non-turning rotation is detected as the noise, detect a turn from the rotation from which the noise has been removed, and if no non-turning rotation has been detected, detecting the turn of the user based on the rotation; and

generating information regarding the turn,

wherein the sensor includes a sensor mounted on a head on the user or a piece of equipment mounted on the head of the user,

wherein the non-turning rotation includes a rotation occurring through a head shake of the user, and

wherein the sensor data analysis unit is configured to detect the head shake of the user when clockwise and counterclockwise rotations are consecutively performed at a time interval that is smaller than or equal to a threshold value.

12. A program causing a computer to perform the method of claim 11.

**Patentansprüche**

1. Informationsverarbeitungsvorrichtung (100) umfassend:

eine Sensordatenerfassungseinheit, die dazu ausgelegt ist, Sensordaten zu erfassen, die von einem Sensor bereitgestellt werden, der von einem Benutzer getragen wird oder an einer vom Benutzer verwendeten Ausrüstung angebracht ist;

eine Sensordatenanalyseeinheit, die dazu ausgelegt ist, eine Drehung des Benutzers auf der Grundlage der Sensordaten zu erkennen, wobei das Erkennen der Drehung das Erkennen einer Rotation in einer Handlung des Benutzers, das Erkennen einer nicht-drehenden Rotation als Rauschen in Bezug auf die zu erkennende Drehung, die in den Sensordaten beinhaltet ist, das Bestimmen, ob eine nicht-drehende Rotation erkannt wurde, beinhaltet, wobei, wenn eine nicht-drehende Rotation als das Rauschen erkannt wird, die Drehung aus der Rotation, aus der das Rauschen entfernt wurde, zu erkennen, und, wenn keine nicht-drehende Rotation erkannt wurde, Erkennen der Drehung des Benutzers basierend auf der Rotation; und

eine Informationserzeugungseinheit, die dazu ausgelegt ist, Informationen bezüglich der Drehung zu erzeugen;

wobei der Sensor einen am Kopf des Benutzers angebrachten Sensor oder eine am Kopf des Benutzers angebrachte Ausrüstung beinhaltet,

wobei die nicht-drehende Rotation eine Rotation beinhaltet, die durch ein Kopfschütteln des Benutzers entsteht, und

wobei die Sensordatenanalyseeinheit dazu ausgelegt ist, das Kopfschütteln des Benutzers zu erkennen, wenn die Rotationen im Uhrzeigersinn und gegen den Uhrzeigersinn nacheinander in einem Zeitintervall durchgeführt werden, das kleiner oder gleich einem Schwellenwert ist.

2. Informationsverarbeitungsvorrichtung (100) nach Anspruch 1,

wobei der Sensor einen Beschleunigungssensor und einen Winkelgeschwindigkeitssensor beinhaltet, und die Handlungserkennungseinheit die Handlung auf der Grundlage eines aus den Sensordaten extrahierten Merkmalsbetrags erkennt, oder, ferner umfassend:

EP 3 243 554 B1

eine Punkteverarbeitungseinheit (109), die dazu ausgelegt ist, eine Punktezahl für das Bewerten der Drehung zu berechnen,
wobei die Informationserzeugungseinheit auf der Grundlage der Punktezahl Informationen bezüglich der Drehung erzeugt.

3. Informationsverarbeitungsvorrichtung (100) nach Anspruch 2, wobei die Informationserzeugungseinheit Informationen erzeugt, die eine auf der Grundlage der Punktezahl geschätztes Fähigkeitsniveaus der Drehung des Benutzers beinhalten.

4. Informationsverarbeitungsvorrichtung (100) nach Anspruch 3, wobei die Informationserzeugungseinheit Informationen einschließlich einer Rangfolge erzeugt, die sich auf eine Fähigkeit der Drehung des Benutzers bezieht, die auf der Grundlage des Fähigkeitsniveaus entschieden wird, und/oder, wobei die Informationserzeugungseinheit Informationen einschließlich einer Bewertung jeder Einrichtung erzeugt, an der die Drehung ausgeführt wird, die auf der Grundlage des Fähigkeitsniveaus entschieden wird.

5. Informationsverarbeitungsvorrichtung (100) nach Anspruch 3,

wobei der Benutzer einen ersten und einen zweiten Benutzer beinhaltet, und
die Informationserzeugungseinheit Informationen bezüglich der Drehung durch Vergleichen des ersten Benutzers und des zweiten Benutzers in Bezug auf das Fähigkeitsniveau erzeugt.

6. Informationsverarbeitungsvorrichtung (100) nach Anspruch 5, wobei die Informationserzeugungseinheit Informationen zum Benachrichtigen des ersten Benutzers über die Anwesenheit des zweiten Benutzers erzeugt, wenn das Fähigkeitsniveau des ersten Benutzers nahe dem Fähigkeitsniveau des zweiten Benutzers liegt, insbesondere wobei die Informationserzeugungseinheit die Informationen zum Benachrichtigen des ersten Benutzers über die Anwesenheit des zweiten Benutzers erzeugt, wenn die Positionen des ersten Benutzers und des zweiten Benutzers nahe beieinander liegen.

7. Informationsverarbeitungsvorrichtung (100) nach Anspruch 2, wobei die Punkteverarbeitungseinheit Statistiken über eine Dauer, eine Winkelverschiebung, eine Winkelgeschwindigkeit oder eine Winkelbeschleunigung der Drehung auswertet.

8. Informationsverarbeitungsvorrichtung (100) nach Anspruch 1,

wobei die Handlung die Drehung und einen Sprung oder ein Wackeln beinhaltet, und
die Informationserzeugungseinheit Informationen bezüglich der Drehung und des Sprungs oder des Wackelns erzeugt.

9. Informationsverarbeitungsvorrichtung (100) nach Anspruch 8, ferner umfassend:

eine Punkteverarbeitungseinheit (109), die dazu ausgelegt ist, eine Punktezahl für das Bewerten des Sprungs zu berechnen,
wobei die Punkteverarbeitungseinheit (109) eine Dauer des Sprungs, eine Winkelverschiebung von Sprüngen, Grade des freien Falls der Sprünge und einen Aufprall zum Zeitpunkt eines Starts oder einer Landung des Sprungs auswertet,
oder ferner umfassend:

eine Punkteverarbeitungseinheit (109), die dazu ausgelegt ist, eine Drehungspunktezahl zur Bewertung der Drehung und eine Sprungpunktezahl zur Bewertung des Sprungs zu berechnen,
wobei die Informationserzeugungseinheit Informationen erzeugt, die ein Fähigkeitsniveau des Benutzers beinhalten, das auf der Grundlage der Drehungspunktezahl und der Sprungpunktezahl geschätzt wird.

10. Informationsverarbeitungsvorrichtung (100) nach Anspruch 1, wobei die Informationserzeugungseinheit Informationen zum Benachrichtigen des Benutzers über das Vorhandensein eines Videos, das die Drehung aufnimmt, erzeugt, und/oder wobei die Informationserzeugungseinheit Informationen zum Visualisieren eines Betrags der Drehung in einer Zeitserienweise erzeugt.

11. Informationsverarbeitungsverfahren, umfassend:

Erfassen von Sensordaten, die von einem Sensor bereitgestellt werden, der von einem Benutzer getragen wird oder an einer vom Benutzer verwendeten Ausrüstung angebracht ist;

Erkennen einer Handlung des Benutzers durch einen Prozessor auf der Grundlage der Sensordaten, wobei die Handlung eine Drehung beinhaltet,

wobei der Prozessor ferner eine Drehung des Benutzers auf der Grundlage der Sensordaten erkennt, wobei das Erkennen der Drehung das Erkennen einer Rotation in einer Handlung des Benutzers, das Erkennen einer nicht-drehenden Rotation als Rauschen in Bezug auf die zu erkennende Drehung, die in den Sensordaten beinhaltet ist, das Bestimmen, ob eine nicht-drehende Rotation erkannt wurde, beinhaltet, wobei, wenn eine nicht-drehende Rotation als das Rauschen erkannt wird, eine Drehung aus der Rotation zu erkennen, aus der das Rauschen entfernt wurde, und, wenn keine nicht-drehende Rotation erkannt wurde, Erkennen der Drehung des Benutzers basierend auf der Rotation; und

Erzeugen von Informationen bezüglich der Drehung,

wobei der Sensor einen an einem Kopf des Benutzers angebrachten Sensor oder eine am Kopf des Benutzers angebrachte Ausrüstung beinhaltet,

wobei die nicht-drehende Rotation eine Rotation beinhaltet, die durch ein Kopfschütteln des Benutzers entsteht, und

wobei die Sensordatenanalyseeinheit dazu ausgelegt ist, das Kopfschütteln des Benutzers zu erkennen, wenn die Rotationen im Uhrzeigersinn und gegen den Uhrzeigersinn nacheinander in einem Zeitintervall durchgeführt werden, das kleiner oder gleich einem Schwellenwert ist.

12. Programm, das einen Computer veranlasst, das Verfahren nach Anspruch 11 durchzuführen.


**Revendications**

1. Dispositif de traitement d'informations (100) comprenant :

une unité d'acquisition de données de capteur configurée pour acquérir des données de capteur fournies par un capteur porté par un utilisateur ou monté sur un équipement utilisé par l'utilisateur ;

une unité d'analyse de données de capteur configurée pour détecter un virage de l'utilisateur en fonction des données de capteur, la détection du virage incluant la détection d'une rotation dans une action de l'utilisateur, la détection d'une rotation sans virage en tant que bruit par rapport au virage à détecter inclus dans les données de capteur, la détermination du fait qu'une rotation sans virage a ou non été détectée, où, si une rotation sans virage est détectée en tant que bruit, un virage est détecté à partir de la rotation depuis laquelle le bruit a été retiré, et si aucune rotation sans virage n'est détectée, un virage de l'utilisateur est détecté en fonction de la rotation ; et

une unité de génération d'informations configurée pour générer des informations concernant le virage ;

le capteur comprenant un capteur monté sur la tête de l'utilisateur ou sur un équipement monté sur la tête de l'utilisateur,

la rotation sans virage comprenant une rotation survenant lorsque l'utilisateur secoue la tête, et

l'unité d'analyse de données de capteur étant configurée pour détecter que l'utilisateur secoue la tête lorsque des rotations dans le sens des aiguilles d'une montre et dans le sens inverse sont effectuées de manière consécutive sur un intervalle de temps qui est inférieur ou égal à une valeur seuil.

2. Dispositif de traitement d'informations (100) selon la revendication 1,

le capteur comprenant un capteur d'accélération et un capteur de vitesse angulaire, et

l'unité de détection d'action détectant l'action en fonction d'une quantité de caractéristiques extraites des données du capteur, ou comprenant en outre :

une unité de traitement de score (109) configurée pour calculer un score pour l'évaluation du virage, l'unité de génération d'informations générant des informations concernant le virage en fonction du score.

3. Dispositif de traitement d'informations (100) selon la revendication 2, dans lequel l'unité de génération d'informations génère des informations incluant un niveau de dextérité de l'utilisateur pour le virage, estimé en fonction du score.

4. Dispositif de traitement d'informations (100) selon la revendication 3, dans lequel l'unité de génération d'informations génère des informations incluant un classement concernant une dextérité de l'utilisateur pour le virage, qui est décidé en fonction du niveau de dextérité, et/ou dans lequel l'unité de génération d'informations génère des informations incluant une évaluation de chaque installation au sein de laquelle le virage est exécuté, qui est décidée en fonction du niveau de dextérité.

**5.** Dispositif de traitement d'informations (100) selon la revendication 3,

dans lequel l'utilisateur inclut un premier utilisateur et un deuxième utilisateur, et
l'unité de génération d'informations génère des informations concernant le virage en comparant les niveaux de dextérité du premier utilisateur et du deuxième utilisateur.

**6.** Dispositif de traitement d'informations (100) selon la revendication 5, dans lequel l'unité de génération d'informations génère des informations pour notifier au premier utilisateur la présence du deuxième utilisateur lorsque le niveau de dextérité du premier utilisateur est proche du niveau de dextérité du deuxième utilisateur, en particulier, l'unité de génération d'informations générant les informations pour notifier au premier utilisateur la présence du deuxième utilisateur lorsque les positions du premier utilisateur et du deuxième utilisateur sont proches.

**7.** Dispositif de traitement d'informations (100) selon la revendication 2, dans lequel l'unité de traitement de score évalue des statistiques d'une durée, d'un déplacement angulaire, d'une vitesse angulaire, ou d'une accélération angulaire du virage.

**8.** Dispositif de traitement d'informations (100) selon la revendication 1,

dans lequel l'action inclut le virage et un saut ou une chute, et
l'unité de génération d'informations génère des informations concernant le virage et le saut ou la chute.

**9.** Dispositif de traitement d'informations (100) selon la revendication 8, comprenant en outre :

une unité de traitement de score (109) configurée pour calculer un score pour l'évaluation du saut,
l'unité de traitement de score (109) évaluant une durée du saut, un déplacement angulaire des sauts, des degrés de chute libre des sauts, et un impact au moment d'un décollage ou d'un atterrissage du saut, ou, comprenant en outre :

une unité de traitement de score (109) configurée pour calculer un score de virage pour évaluer le virage et un score de saut pour évaluer le saut,
l'unité de génération d'informations générant des informations incluant un niveau de dextérité de l'utilisateur estimé en fonction du score de virage et du score de saut.

**10.** Dispositif de traitement d'informations (100) selon la revendication 1, dans lequel l'unité de génération d'informations génère des informations pour notifier à l'utilisateur la présence d'une capture vidéo du virage et/ou dans lequel l'unité de génération d'informations génère des informations pour la visualisation d'une partie du virage sous forme d'une série chronologique.

**11.** Procédé de traitement d'informations comprenant :

l'acquisition de données de capteur fournies par un capteur porté par un utilisateur ou monté sur un équipement utilisé par l'utilisateur ;
la détection, par un processeur, d'une action de l'utilisateur en fonction des données de capteur, l'action incluant un virage,
un processeur détectant en outre un virage de l'utilisateur en fonction des données de capteur, la détection du virage incluant la détection d'une rotation dans une action de l'utilisateur, la détection d'une rotation sans virage en tant que bruit par rapport au virage à détecter inclus dans les données de capteur, la détermination du fait qu'une rotation sans virage a ou non été détectée, où, si une rotation sans virage est détectée en tant que bruit, un virage est détecté à partir de la rotation depuis laquelle le bruit a été retiré, et si aucune rotation sans virage n'est détectée, un virage de l'utilisateur est détecté en fonction de la rotation ; et
la génération d'informations concernant le virage,
le capteur comprenant un capteur monté sur la tête de l'utilisateur ou sur un équipement monté sur la tête de l'utilisateur,
la rotation sans virage incluant une rotation se produisant lorsque l'utilisateur secoue la tête, et l'unité d'analyse de données de capteur étant configurée pour détecter que l'utilisateur secoue la tête lorsque des rotations dans le sens des aiguilles d'une montre et dans le sens inverse sont effectuées de manière consécutive sur un intervalle de temps qui est inférieur ou égal à une valeur seuil.

12. Programme faisant en sorte qu'un ordinateur exécute le procédé selon la revendication 11.

# FIG. 1

EP 3 243 554 B1

# FIG. 2

```
                    ┌─────────────────────┐
                    │  JUMP Detection 1   │
                    │       START         │
                    └─────────────────────┘
```

| HIGH IMPACT Detection ~S110 | | FREE FALL Detection ~S120 |

Impact-Impact Section? ~S101 — NO

YES

TH1 < Section Duration < TH2? ~S102 — NO

YES

Free Fall Rate > TH? ~S103 — NO

YES

Detect JUMP Section ~S104

END

# FIG. 3

```
        ╭─────────────────────────╮
        │   HIGH IMPACT Detection │
        │         START           │
        ╰─────────────────────────╯
                     │
                     ▼
        ╱─────────────────────────╱ ──D111
       ╱      Acceleration       ╱
      ╱─────────────────────────╱
                     │
                     ▼
        ┌─────────────────────────┐
        │          Norm           │ ──S112
        └─────────────────────────┘
                     │
                     ▼
        ┌─────────────────────────┐
        │          LPF            │ ──S113
        └─────────────────────────┘
                     │
                     ▼
        ┌─────────────────────────┐
        │         Power           │ ──S114
        └─────────────────────────┘
                     │               ──S115
                     ▼
              ╱───────────╲              NO
            ╱   Power > TH?  ╲───────────────┐
              ╲───────────╱                  │
                     │                       │
                    YES                      │
                     ▼                       │
        ┌─────────────────────────┐          │
        │ Detect HIGH IMPACT Section│ ──S116  │
        └─────────────────────────┘          │
                     │◄──────────────────────┘
                     ▼
              ╭─────────────╮
              │     END     │
              ╰─────────────╯
```

# FIG. 4

FREE FALL Detection 1
START

Acceleration ⟶ D121

Norm ⟶ S122

S123
Norm < TH? — NO

YES

S124
Detect FREE FALL Section

Angular Rate ⟶ D125

Norm ⟶ S126

Variance ⟶ S127

S128
Variance < TH? — NO

YES

Mask ⟶ S129

END

# FIG. 5

```
        ┌──────────────────────┐
        │  FREE FALL Detection 2│
        │       START          │
        └──────────┬───────────┘
                   │
         ┌─────────▼─────────┐
        ╱   Acceleration     ╱──D121
       └──────────┬──────────┘
                  │
        ┌─────────▼─────────┐
        │       Norm        │──S122
        └─────────┬─────────┘
                  │          S123
              ◇───▼───◇          NO
             ╱ Norm < TH? ╲─────────┐
              ◇───┬───◇              │
                YES    S124          │
        ┌─────────▼─────────────┐    │
        │ Detect FREE FALL Section│   │
        └─────────┬─────────────┘    │
                  │◄─────────────────┘
                  │
```

```
                   ┌──────────────────────┐
                   │                      │
          ┌────────▼─────────┐
         ╱   Acceleration     ╱──D121
        └─────────┬──────────┘
                  │
        ┌─────────▼─────────┐
        │       X, Y        │──S132
        └─────────┬─────────┘
                  │
        ┌─────────▼─────────┐
        │    Covariance     │──S133
        └─────────┬─────────┘
                  │           S134
              ◇───▼───◇           NO
             ╱Covariance < TH?╲────────┐
              ◇───┬───◇                 │
                YES                     │
        ┌─────────▼─────────┐           │
        │       Mask        │──S129     │
        └─────────┬─────────┘           │
                  │◄────────────────────┘
                  │
           ┌──────▼──────┐
           │     END     │
           └─────────────┘
```

# FIG. 6

```
         ┌─────────────────────┐
         │   JUMP Detection 2   │
         │       START         │
         └─────────────────────┘
                    │
                    ▼
      ┌─────────────────────────┐
      │    Candidate Section    │ ～S140
      │       Detection         │
      └─────────────────────────┘
                    │
                    ▼          ～S105
              ◇─────────────────◇          NO
             ╱  Candidate Section? ╲──────────────┐
              ◇─────────────────◇                 │
                    │ YES                          │
                    ▼          ～S102              │
              ◇─────────────────◇          NO      │
             ╱ TH1 < Section Duration < TH? ╲──────┤
              ◇─────────────────◇                 │
                    │ YES                          │
                    ▼          ～S106              │
              ◇─────────────────◇          NO      │
             ╱ Acceleration Means > THs? ╲─────────┤
              ◇─────────────────◇                 │
                    │ YES                          │
                    ▼                              │
      ┌─────────────────────────┐                 │
      │    Detect JUMP Section   │ ～S104          │
      └─────────────────────────┘                 │
                    │                              │
                    │◄─────────────────────────────┤
                    │◄─────────────────────────────┘
                    ▼
              ┌──────────┐
              │   END    │
              └──────────┘
```

# FIG. 7

```
        ┌──────────────────────────┐
        │ Candidate Section Detection │
        │          START           │
        └──────────────────────────┘
                     │
 ════════════════════╪═══════════════════════════════════════════
        │                    │                        │
       ~S110               ~S141                    ~S142
 ┌──────────────┐  ┌──────────────────┐   ┌──────────────────────┐
 │  HIGH IMPACT │  │ Vertical Acceleration │ │ Horizontal Acceleration │
 │   Detection  │  │       Calc.       │   │         Calc.         │
 └──────────────┘  └──────────────────┘   └──────────────────────┘
        │                    └──────────┬────────────┘
        │                         ┌──────────┐
        │                         │ Difference │~S143
        │                         └──────────┘
        │                              │
 ═══════╪══════════════════════════════╪═══════════════════════════
        └──────────────┬───────────────┘
                       │
                      ~S144
              ◇─────────────────────◇   NO
              │ Impact-Impact Section? │──────┐
              ◇─────────────────────◇       │
                       │ YES                 │
                      ~S145                  │
              ◇─────────────────────◇   NO  │
              │    Difference > TH?    │─────┤
              ◇─────────────────────◇      │
                       │ YES                │
              ┌──────────────────────┐     │
              │ Detect Candidate Section │~S146 │
              └──────────────────────┘     │
                       │◄────────────────────┘
                       │◄─────────────────────┘
                       │
                 ┌──────────┐
                 │   END    │
                 └──────────┘
```

# FIG. 8

```
        ┌─────────────────────────────┐
        │  Vertical Acceleration Calc. │
        │           START              │
        └─────────────────────────────┘
                      │
                      ▼
        ┌─────────────────────────────┐
        │        Acceleration          │──── D151
        └─────────────────────────────┘
            │                     │
            ▼ ~S152               ▼ ~S155
    ┌───────────────┐     ┌───────────────┐
    │     Mean      │     │      BPF       │
    └───────────────┘     └───────────────┘
            │ ~S153               │ ~S156
            ▼                     ▼
    ┌───────────────┐     ┌───────────────┐
    │Gravity        │- - >│ Inner Product  │
    │Acceleration   │     │                │
    │Calc.          │     │                │
    └───────────────┘     └───────────────┘
            │ ~S154               │
            ▼                     │
    ┌───────────────┐             │
    │     Norm      │             │
    └───────────────┘             │
            │                     │
            └──────────┬──────────┘
                       ▼
        ┌─────────────────────────────┐
        │          Division            │── S157
        └─────────────────────────────┘
                      │
                      ▼
        ┌─────────────────────────────┐
        │     Vertical Acceleration    │── V158
        └─────────────────────────────┘
                      │
                      ▼
                ┌───────────┐
                │    END     │
                └───────────┘
```

# FIG. 9

```
        ┌─────────────────────────────┐
        │ Horizontal Acceleration Calc.│
        │          START              │
        └─────────────────────────────┘
                      │
                      ▼
        ╱─────────────────────────────╱─── D151
       ╱        Acceleration         ╱
      ╱─────────────────────────────╱
            │                    │
            ▼ ~S141              ▼ ~S162
   ┌║──────────────────║┐   ┌──────────────────┐
   ┌║Vertical Acceleration║┐  │       BPF        │
   ┌║     Calc.         ║┐  └──────────────────┘
   └║──────────────────║┘           │
            │                       ▼ ~S163
            │              ┌──────────────────┐
            │              │       Norm        │
            │              └──────────────────┘
            ▼ ~S161                 │
   ┌──────────────────┐            ▼ ~S164
   │     Square        │   ┌──────────────────┐
   └──────────────────┘   │      Square       │
            │              └──────────────────┘
            └──────┐    ┌──────┘
                   ▼    ▼
        ┌─────────────────────────────┐
        │        Difference           │─── S165
        └─────────────────────────────┘
                      │
                      ▼
        ┌─────────────────────────────┐
        │           Root              │─── S166
        └─────────────────────────────┘
                      │
                      ▼
        ┌─────────────────────────────┐
        │   Horizontal Acceleration   │─── V167
        └─────────────────────────────┘
                      │
                      ▼
                ┌───────────┐
                │    END    │
                └───────────┘
```

# FIG. 10

```
        ┌──────────────────────┐
        │    TURN Detection     │
        │        START          │
        └──────────┬───────────┘
                   │
                   ▼
        ┌──────────────────────┐
        │   ROTATION Section    │───── S210
        │      Detection        │
        └──────────┬───────────┘
                   │
                   ▼          ╱──── S201
              ╱─────────────╲
             ╱  ROTATION     ╲       NO
             ╲  Section?      ╱──────────────┐
              ╲─────────────╱               │
                   │ YES                     │
                   ▼                         │
        ┌──────────────────────┐            │
        │     HEAD SHAKE        │───── S230  │
        │      Detection        │            │
        └──────────┬───────────┘            │
                   │          ╱──── S203      │
                   ▼                         │
              ╱─────────────╲                │
             ╱  HEAD SHAKE   ╲     YES        │
             ╲  Detected?     ╱───────────┐  │
              ╲─────────────╱             │  │
                   │ NO                    │  │
                   ▼                       │  │
        ┌──────────────────────┐          │  │
        │    TURN Detection     │── S250   │  │
        │                       │          │  │
        └──────────┬───────────┘          │  │
                   │◄─────────────────────┘  │
                   │◄────────────────────────┘
                   ▼
              ┌─────────┐
              │   END   │
              └─────────┘
```

# FIG. 11

```
                    ROTATION Section Detection
                           START

         ┌──────────────────────┴──────────────────────┐
         ▼                                              ▼
    ╱ Acceleration ╲──D211          ╱ Angular Rate ╲──D214
         │                                   │
         ▼  S212                             │
    ┌──────────────┐                         │
    │     Mean     │                         │
    └──────────────┘                         │
         │  S213                             │
    ┌──────────────┐                         │
    │   Gravity Acceleration                 │
    │      Calc.   │                         │
    └──────────────┘                         │
         │  S215                             │
    ┌──────────────┐◄────────────────────────┘
    │ Inner Product│
    └──────────────┘
         │  V216
    ┌──────────────┐
    │  Horizontal  │
    │ Angular Rate │
    └──────────────┘
         │  S217
    ┌──────────────┐
    │   Integral   │
    └──────────────┘
         │  V218
    ┌──────────────┐
    │Horizontal Angle│
    └──────────────┘
         │
```

```
         ┌──────────────────────────────────────┐
         ▼                                  S219
    ┌──────────────┐
    │     LPF      │
    └──────────────┘
         │  S220
    ┌──────────────┐
    │ Differentiate│
    └──────────────┘
         │  V221
    ┌──────────────┐
    │  Horizontal  │
    │ Angular Rate │
    └──────────────┘
         │  S222
    ◇ Angular Rate > TH? ◇───NO───┐
         │  YES                    │
         │  S223                   │
    ┌──────────────────────┐       │
    │Detect ROTATION Section│      │
    └──────────────────────┘       │
         │◄──────────────────────── ┘
         ▼
        END
```

# FIG. 12

HEAD SHAKE Detection
START

Horizontal
Angular Rate — V221

Sign — S231

Clockwise Rotation — V232

Counterclockwise
Rotation — V233

Time Interval Calc. — S234

Time Interval < TH? — S235

NO

YES

Detect HEAD SHAKE — S236

END

## FIG. 13

EP 3 243 554 B1

**FIG. 14**

EP 3 243 554 B1

**FIG. 15**

```
                    ( START )
                        │
                        ▼
        ┌──────────────────────────────┐
        │    DETECT ACTION SECTION      │──S301
        └──────────────────────────────┘
                        │
                        ▼
        ┌──────────────────────────────┐
        │   CALCULATE ACTION SCORE      │──S302
        └──────────────────────────────┘
                        │
                        ▼
        ┌──────────────────────────────┐
        │        UPLOAD DATA            │──S303
        └──────────────────────────────┘
                        │
                        ▼
        ┌──────────────────────────────┐
        │     CALCULATE SKILL LEVEL     │──S304
        └──────────────────────────────┘
```

S305

| SEARCH FOR SKILL LEVEL BY USING USER ID |

S307
| CALCULATE RANKING INFORMATION |

S311
| SEARCH FOR FACILITY ID BY USING POSITION INFORMATION |

S309
| DECIDE USER WITH WHOM VIDEO WILL BE SHARED |

S312
| CALCULATE FACILITY LEVEL |

S306
| PROVIDE SKILL LEVEL INFORMATION |

S308
| PROVIDE RANKING INFORMATION |

S310
| ACQUIRE VIDEO DATA |

S313
| PROVIDE FACILITY DATA |

S314
| SHARE |

( END )

EP 3 243 554 B1

## FIG. 16

**Profile** — 1000

ID: Jowel — 1001
Skill Lv: Super Beginner — 1003
Skill Pt: 65,536 pt — 1005
Last Play: 11/Jan/2015

**Personal Data**

- 11/Jan/2015

@ South Park
Action Time: 01:20
Total Jump: 5times
Total Turn: 2times

- 31/Dec/2014

@ West Village
Action Time: 02:00
Total Jump: 2times
Total Turn: 6times — 1017

**Movie**

Jump @ South Park
11/Jan/2015 — 1015

◀◀  ▶  ■  ▶▶

Jump @ West Village
31/Dec/2014

**West Village** / 35.65, 139.74 — 1021
Place Lv: Beginner
Place Pt: 123,456 pt
Player : 95 — 1019

**Skill Ranking** — 1007

| Total | Area | Place | Age | Type | — 1009, 1011, 1013

1st  ID: Kent
     Skill Lv: Top Player
     Skill Pt: 859,213 pt

2nd  ID: Sara
     Skill Lv: Top Player
     Skill Pt: 621,004 pt

3rd  ID: Brian
     Skill Lv: Semi Top Player
     Skill Pt: 590,981 pt

4th  ID: Sidy Skill Lv: Ultra Player

5th  ID: Arial Skill Lv: Ultra Player

6th  ID: Gosh Skill Lv: Super Player
     ⋮

EP 3 243 554 B1

# FIG. 17

```
        START
          │
          ▼
  DETECT ACTION SECTION ──S301
          │
          ▼
  CALCULATE ACTION SCORE ──S302
          │
          ▼
     UPLOAD DATA ──S303
          │
          ▼
  CALCULATE SKILL LEVEL ──S304
          │
  ┌───────┴───────┐
  ▼               ▼
S321            S322
EXECUTE USER    EXECUTE USER
SEARCH BASED    SEARCH BASED
ON IMAGE        ON POSITION
MATCHING        AND BEARING
  └───────┬───────┘
          ▼
  ACQUIRE TARGET USER ID ──S323
          │
  ┌───────┼───────────────┐
  ▼       ▼               ▼
S324     S326            S328
COMPARE  ACQUIRE SKILL   ACQUIRE
SKILL    LEVEL           VIDEO DATA
LEVELS   INFORMATION
  │       └───────┬───────┘
  ▼               ▼
S325            S327
NOTIFY          PERFORM DISPLAY
                ON HMD
  └───────┬───────┘
          ▼
         END
```

**FIG. 18**

R

1100a

1101
Your rival is appeared!

R

1103
Go straight 50m, then turn left.

1100b

Your rival is here!

N

1107

1105

S

1109

**FIG. 19**

**FIG. 20**

# FIG. 21

START

DETECT ACTION SECTION — S301

ISSUE VIDEO RECEPTION REQUEST OF ACTION SECTION — S341

DECIDE TARGET CAMERA ON BASIS OF POSITION INFORMATION — S342

SEARCH VIDEO DATA FOR ACTION SECTION — S343

DOES DATA EXIST? — S344

CREATE THUMBNAIL IMAGE — S345

TRANSMIT THUMBNAIL IMAGE — S346

CHECK DOWNLOADING — S347

CONSENTED? — S348    NO
YES

PROCESS VIDEO — S349

TRANSMIT VIDEO — S350

HAS VIDEO RECEPTION BEEN COMPLETED? — S351    NO
YES

PERFORM BILLING PROCESS — S352

END

FIG.22

# FIG. 23

# FIG. 24

START

↓

DETECT ACTION SECTION — S301

↓

| CALCULATE ACTION SCORE — S302 | GENERATE VISUALIZATION DATA — S381 | SYNCHRONIZE SENSOR DATA WITH BIOLOGICAL SENSOR INFORMATION — S382 |

↓

UPLOAD DATA — S383

↓

| GENERATE COACHING INFORMATION — S384 | GENERATE COMPARATIVE DATA — S385 |

↓

REQUEST — S386

↓

PROVIDE DATA — S387

↓

END

EP 3 243 554 B1

**FIG. 25**

## FIG. 26

EP 3 243 554 B1

EP 3 243 554 B1

# FIG. 27

**FIG. 28**

EP 3 243 554 B1

# FIG. 29

FIG. 30

EP 3 243 554 B1

# FIG.31

## FIG.32

# FIG. 33

# FIG. 34

EP 3 243 554 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130044043 A1
- US 2013330054 A1
- JP 2010198595 A
- WO 2014125689 A